# EUROPEAN PATENT SPECIFICATION

(11) **EP 2 171 448 B1**
(45) Date of publication and mention of the grant of the patent: **15.08.2012**
(21) Application number: 08768379.3
(22) Date of filing: 12.06.2008
(51) Int. Cl.: G01N 33/50, A61P 35/00, A61K 31/00, A61K 39/00, A61K 48/00

(54) **METHODS FOR INHIBITING TUMOR GROWTH BY TARGETING THE SSDNA REPLICATION INTERMEDIATE OF TUMOR STEM CELLS**
VERFAHREN ZUR HEMMUNG VON TUMORWACHSTUM UNTER ABZIELEN AUF DIE SSDNA-REPLIKATIONSZWISCHENSTUFE VON TUMORSTAMMZELLEN
MÉTHODES POUVANT INHIBER UNE CROISSANCE TUMORALE CIBLANT L'ETAT INTERMEDIAIRE DE REPLICATION A l'ADN SIMPLE BRIN DES CELLULES SOUCHES TUMORALES

(30) Priority: 13.06.2007 US 934420 P
(43) Date of publication of application: 07.04.2010
(73) Proprietor: Massachusetts Institute of Technology, Cambridge, MA 02139 (US)
(72) Inventor: GOST JEVA, Elena, V., Winchester, MA 01890 (US); THILLY, William, G., Winchester, MA 01890 (US)
(74) Representative: Baldock, Sharon Claire
(86) International application number: PCT/US2008/007327
(87) International publication number: WO 2008/156629

(56) References cited:
- WO-A-2007/067795
- GOSTJEVA ET AL: "Bell-shaped nuclei dividing by symmetrical and asymmetrical nuclear fission have qualities of stem cells in human colonic embryogenesis and carcinogenesis" CANCER GENETICS AND CYTOGENETICS, ELSEVIER SCIENCE PUBLISHING, NEW YORK, NY, US, vol. 164, no. 1, 1 January 2006 (2006-01-01), pages 16-24, XP005214800 ISSN: 0165-4608
- WADKINS R M ET AL: "Actinomycin D binds to metastable hairpins in single-stranded DNA." BIOCHEMISTRY 25 AUG 1998, vol. 37, no. 34, 25 August 1998 (1998-08-25), pages 11915-11923, XP002491351 ISSN: 0006-2960
- WHITE R J ET AL: "Development of mitoxantrone." INVESTIGATIONAL NEW DRUGS 1985, vol. 3, no. 2, 1985, pages 85-93, XP009104311 ISSN: 0167-6997
- AL-HAJJ M ET AL: "Self-renewal and solid tumor stem cells", ONCOGENE, NATURE PUBLISHING GROUP, GB, vol. 23, no. 43, 20 September 2004 (2004-09-20), pages 7274-7282, XP002380287, ISSN: 0950-9232, DOI: DOI:10.1038/SJ.ONC.1207947
- GOSTJEVA ELENA V ET AL: "Stem cell stages and the origins of colon cancer: a multidisciplinary perspective", STEM CELL REVIEWS AND REPORTS, HUMANA PRESS INC, US, vol. 1, no. 3, 1 January 2005 (2005-01-01) , pages 243-251, XP009104247, ISSN: 1550-8943, DOI: DOI:10.1385/SCR:1:3:243

## Description

### BACKGROUND OF THE INVENTION

Scientists have recognized the resemblance of tumor cells and pathological tissue constructs (such as teratocarcinomas) to the cells and tissues of early embryos. Normal but undifferentiated embryonic stem cells were able to give rise to organs by undefined processes that logically had to include rapid increase in cell number and differentiation to in organ anlage and subsequent organogenesis. Malignant tumors grow at rates similar to early fetuses and contain niches that are either histologically undifferentiated or organized with a histological appearance of normal tissue.

Tumors and fetal tissues appear to share many molecules and processes in common. For example, complex antigenic glycosoaminoglycans at the cell surface, the "carcinoembryonic antigens," are expressed in both fetal tissues and tumors, as are cell adhesion molecules. Oncogenesis like ontogenesis appears to proceed by lineal descent through an expanding set of stem cells. Only a small fraction of cells from a human tumor have the capacity, alone or in concert with other cells, to form new tumors as xenografts in immuno-suppressed rodents. Limiting dilution xenograft experiments have shown that one or more cells among the putative tumorigenic cells display stem cell-like properties in that they are capable of generating new tumors containing additional stem cells as well as regenerating the phenotypically mixed populations of cells present in the original tumor.

The concept of monoclonality of tumors was established in the early 1900's. More recently, it was determined that nearly all forms of late-onset cancer pass through an extended period of preneoplasia and that these preneoplastic colonies were themselves monoclonal and resulting from more than one rare cytogenetic mutation from the germinal DNA. By the beginning of 2 1 st century, direct attempts to enrich tumor cell populations with "stem" cells for transplant/dilution experiments had demonstrated that not only were tissue stem cells the likely origin of preneoplasia, but tumors themselves contained "stem" cells. Modern restatement of the hypothesis that tumors are in fact reasonably well-organized heterogeneous fetal structures has been suggested. 'Carcinoembryonic' stem cells would be expected to increase in number and give rise to differentiated cell types populating the highly heterogeneous niches within the tumor mass.

Various antigenic markers employed throughout the stem cell field have been used to enrich for cells capable of regenerating tissues or tumors ostensibly to a high degree. No cells within these enriched populations, however, have demonstrated any microscopic morphological cellular property that marks them as stem cells. If it is true that tumors arise from a single stem cell, a means is required to identify them and to collect them as homogeneous population of stem cells sufficient for analysis of molecular and biochemical analytes. Of primary importance is the identification of tumor cell targets for the development of novel therapies directed to selectively inhibiting the growth of, or killing, the stem cells of neoplasias and preneoplasias.

GOSTJEVA ET AL describe "Bell-shaped nuclei dividing by symmetrical and asymmetrical nuclear fission have qualities of stem cells in human colonic embryogenesis and carcinogenesis" in: CANCER GENETICS AND CYTOGENETICS, ELSEVIER SCIENCE PUBLISHING, NEW YORK, NY, US vol 164, no 1, 1 January 2006 pages 16-24.

WADKINS R. M. ET AL describe "Actinomycin D binds to metastable hairpins in single-stranded DNA" in: BIOCHEMISTRY 25 AUGUST 1998, vol 37, no. 34, 25 August 1998 pages 11915-11923.

WHITE R.J. ET AL described "Development of mitoxantrone" in: INVESTIGATIONAL NEW DRUGS 1985, vol 3, no. 2, 1985 pages 85-93.

### SUMMARY OF THE INVENTION

Mutations within stem cell populations are required to generate cancer stem cells. The modem "tumor stem cell" hypothesis derived from 19^{th} century pathological observations postulates that tumors contain unique subsets of cells that have properties similar to both normal stem cells and fetal tissues. These cells proliferate extensively through asymmetric and non-mitotic rounds of cell divisions. This population of cells is likely self-renewing and is responsible for the growth and maintenance of most tumors. As such, the inhibition of tumor stem cell division is widely believed to represent a novel therapeutic intervention that promises improved efficacy over current approaches.

The present invention is based upon observations that indicate that the period of human fetal/juvenile growth is associated with a high rate of mutation in the stem cell lineage and that this lineage practices a previously undetected mode of DNA segregation and replication. Previously described in US Patent Application Number 2006/0063144 (US patent 7,427, 502) and PCT/US06/047136 (International patent, publication no. WO 2007/067795) tumor and fetal stem cells are characterized by a unique nuclear morphotype referred to as bell-shaped nuclei. (Bell-shaped nuclei are also found in syncytia and the methods of the present invention described herein are also understood to encompass bell-shaped nuclei present in syncytia as well as in cells). Tumor and fetal stem cells containing bell-shaped nuclei are referred to herein as metakaryotes. The present invention is based upon the observation of paramount importance, described herein, that the bell-shaped nuclei of tumor and fetal stem cells undergo a unique form of replication. This unique form of genomic replication involves a replicative intermediate with a nuclear structural configuration wherein the bell-shaped nuclei separate (as in a "cup-to-cup" separation), and that during separation a large portion of the genome is first segregated into single stranded DNA (ssDNA) before genomic replication. This observation is in contrast to previous observations of human cell division where DNA synthesis proceeds by iterative copying of short stretches of DNA followed by chromatid condensation and separation at mitosis in double stranded DNA (dsDNA) form.

Furthermore, of equal importance, is the observation that during this unique form of genomic replication a substantial amount of the ssDNA can be observed apart from the ssDNA contained within bell-shaped nuclei, yet this ssDNA is associated with and indicative of the replicative intermediate configuration unique to tumor stem cell replication. Importantly, these key observations, that during the bell-shaped nuclear division, tumor and fetal stem cells and syncytia are in an intermediate configuration where the genome is, for a significant and exploitable period of time, substantially single-stranded DNA (ssDNA), either within or outside of the bell-shaped nuclei, can form the basis for methods of assaying the efficacy of anti-cancer agents and therapies by detecting either bell-shaped nuclei containing ssDNA in the replicative intermediate configuration or by detecting ssDNA alone. Further, this animal (and plant) model of genomic replication unique to tumor and fetal stem cells and syncytia can form the basis of intelligent design of novel cancer therapies by targeting the inhibition or disruption of this stem cell replicative intermediate configuration.

For example, present in this unique tumor stem cell replicative intermediate configuration is a large amount of ssDNA within the bell-shaped nuclei that can be targeted for destruction or degradation resulting in the inhibition of growth, or death of a tumor stem cell. Alternatively, the ssDNA contained in the tumor stem cell but not within the bell-shaped nuclei can also be targeted for destruction or degradation resulting in the inhibition of growth, or death, of a tumor cell. (As used herein, the terms "tumor stem cell" and "metakaryote" are used interchangeably and intended to include both tumor stem cells and fetal stem cells). As used herein, whenever the term "replicative intermediate" or replicative intermediate configuration" is used in conjunction with ssDNA, it is intended to include not only ssDNA contained within the bell-shaped nuclei, but also ssDNA within the tumor stem cell that is not contained within the bell-shaped nuclei. Thus, for example, when agents are described herein that can target ssDNA it is meant that the targeted ssDNA can be contained in the bell-shaped nuclei or alone outside the confines of the bell-shaped nuclei.

It is reasonable to believe that other macromolecules are also present in this replicative intermediate configuration (e.g., a macromolecule required for this replication process is referred to herein as a "tumor stem cell-specific molecule" and this term also encompasses a "fetal stem cell-specific molecule" and a "syncytiaspecific molecule" although not always specifically stated). A tumor stem cell-specific molecule is a molecule present in tumor stem cells, preferably in the nucleus, and not in the surrounding cells (e.g., also referred to herein as a cellular molecule peculiar to the metakaryotic cell). These tumor stem cell-specific molecules (e.g., structural proteins, nucleases, etc.) can also serve as targets for novel anti-cancer therapies.

Thus, the present invention is directed to methods of identifying agents and therapies for the specific inhibition of growth/proliferation, or complete destruction, of tumor stem cells without, or with minimal, damage to normal or maintenance stem cells in their close environment, and to the therapeutic applications of said agents. As described herein, tumor stem cells contain nuclei with unique morphologic bell-shaped characteristics where, during nuclear division, the genome is, for a significant and exploitable period of time, substantially single-stranded DNA (ssDNA). Such unique tumor stem cells, referred to herein as metakaryotes, characteristically contain bell-shaped nuclei comprising, at certain intervals of time, single-stranded DNA. By using agents, *e.g.*, chemical or enzymatic agents, that target and alter the ssDNA of the metakaryotic cell (*e.g.*, alkylating reagents, single-strand-specific nucleases, toxic conjugates), the nuclear material of tumor stem cells is targeted for destruction, as the modified ssDNA would be unable to undergo further replication back into double-stranded DNA (dsDNA).

Also described are methods of identifying targets or markers for tumor stem cells unique to the replicative intermediate configuration of tumor stem cell division. Such targets can be identified for example, by isolating the tumor stem cell containing bell-shaped nuclei undergoing replication, culturing the cells and harvesting the cultured tumor stem cells using standard techniques, and identifying mRNA, transcripts or proteins unique to the replicative intermediates, also by using techniques well know to those of skill in the art. Isolation of the metakaryotes can be based on the presence of bell-shaped nuclei containing ssDNA or ssDNA outside of the nucleus. It is reasonable to believe that the isolation and studying of metakaryotes resulting in the identification of molecules unique to the replicative intermediates described herein would lead to identification of novel targets that would be useful for anti-tumorigenic therapies.

Described herein are *in vitro* and *in vivo* methods for the identification of agents that interact with the unique bell-shaped nuclei replicative intermediate configuration. Such agents can, for example, inhibit or modify tumor, or tumor stem cell, growth by binding to, altering, de-stabilizing, degrading or otherwise modifying ssDNA or other tumor stem cell-specific molecules in the replicative intermediate configuration in the tumor stem cell, thereby inhibiting tumor stem cell replication and/or slowing tumor growth. In particular, a tumor sample (e.g., a tissue biopsy from a solid tumor) will be obtained from a subject, specifically from a solid tumor. The tumor sample can be contacted with a candidate agent under suitable physiologic conditions (e.g., conditions that preserve the bell-shaped nuclei replicative intermediate configuration), and the presence of bell-shaped nuclei in the tumor/tumor sample, in particular the tumor stem cells, will be compared before and after contact/treatment. Agents that inhibit tumor growth will be determined by comparing the number of bell-shaped nuclei containing ssDNA, or the amount of ssDNA alone, in the sample after treatment with the candidate agent to the number of bell-shaped nuclei containing ssDNA or the amount of ssDNA alone in the sample prior to treatment, and to the number of bell-shaped nuclei containing ssDNA or the amount of ssDNA alone in a mock-treated control sample. The amount of ssDNA within bell-shaped nuclei or outside of the bell-shaped nuclei can be qualitatively determined by for example, acridine orange staining, or a quantitative amount can be determined using standard techniques. A reduction in the number of bell-shaped nuclei containing ssDNA, or the absence of bell-shaped nuclei containing ssDNA (or the reduction in amount of, or absence of, ssDNA alone) in the treated tumor or tumor stem cells is indicative of an agent of interest effective in targeting ssDNA and inhibiting the growth of, or killing tumor stem cells. One can also assay for the presence or absence of bell-shaped nuclei replicative intermediates.

Also described is a heterogeneous animal model is created by injecting the animal with a cancer xenograft. Animals suitable for use in an in *vivo* assay method are known to those of skill in the art, and may be specific for the particular type of tumor to be evaluated. For example rodents such as mice and rats are typically used for solid tumor analysis. The method comprises the transplant of the xenograft into the animal, allowing the xenograft to mature into a solid tumor, treating the test animal (and contacting the tumor) with a candidate agent/therapy, excising the tumor and analyzing the tumor tissue for the presence or absence of bell-shaped nuclei replicative intermediates containing ssDNA or ssDNA alone as compared to a control animal (e.g., an animal not treated with the candidate agent). Procedures disclosed in US Patent Application Number 2006/0063144 (US patent 7, 427, 502) can be used to identify bell-shaped nuclear structures. Further following the procedures disclosed in PCT/US06/047136 (International patent, publication no. WO2007/067795) ssDNA within or outside of the bell-shaped nuclei can be identified. For example, the test animal can be treated with agents known to bind, modify, alter or degrade ssDNA (preferentially over double-stranded DNA that is present in the cell) such as alkyalating agents, ssDNA nucleases, other enzymes or proteins. Unlike classic tumor models that look for activity of agents based on the shrinkage of the growing tumor or the survival patterns of the xenotransplanted animal relative to control, this strategy involves assessing the growing xenotransplant for the presence or absence of, or reduction in ssDNA staining metakaryotic cells or ssDNA.

For example, agents of interest (also referred to herein as candidate agents) are agents that significantly reduce the number of acridine orange-staining metakaryotes relative to a control group. (Acridine orange stains single stranded DNA and emits red fluorescence, while under suitable conditions double stranded DNA emits green fluorescence). In another embodiment, agents that prevent reannealing of DNA are used separately or in conjunction with agents that destroy or degrade ssDNA. The assay models of the present invention target the metakaryotic process and thus identifies agents for the treatment, not of tumor bulk, but of the residual surfacing tumor that is not treated with standard agents that target the rapidly dividing mitotic tumor cells.

Also described are methods directed to the therapeutically-selective inhibition of tumor stem cells without substantially preventing or inhibiting the growth of surrounding cells (e.g., maintenance stem cells). For example, while the targeted stem cell is undergoing nuclear division, the method comprises contacting the cell with an agent capable of entering the nucleus of the cell and modifying or altering the bell-shaped nuclei replicative intermediate configuration, by modifying or altering ssDNA resulting in the prevention of further nuclear division of the bell-shaped nuclei in the targeted cell, and/or destruction of the cell. Tumor-stem cell-specific molecules can also be targeted within the cell, whereby targeting and disruption of the function or activity of the tumor stem cell-specific molecule prevents or inhibits tumor cell growth. In particular embodiments, the agent is a chemical agent, radioagent, enzyme, or radiation treatment, whereby the bell-shaped nuclei replicative intermediate is targeted.

Based on the data presented herein, it is reasonable to believe that carcinogenesis appears to be a continuous process in which certain fetal/juvenile stem cells are mutated so they continue growing as juvenile stem cells throughout adult life and acquire such additional change or changes to create a neoplastic stem cell that grows rapidly (fetal rate) and kills. It is upon the recognition of a unique and predominant replicative intermediate in bell-shaped nuclei of human fetuses, preneoplastic and neoplastic lesions that the teaching that the ssDNA in a previously unrecognized form of stem cell DNA replication and segregation, or a stem cell-specific molecule also required for that unique replication process, constitutes a novel valuable target for therapies aiming to kill or slow the growth of the stem cells of human neoplasia and preneoplasia.

### BRIEF DESCRIPTION OF THE DRAWINGS

The patent or application file contains at least one drawing executed in color. Copies of this patent or patent application publication with color drawing(s) will be provided by the Office upon request and payment of the necessary fee.

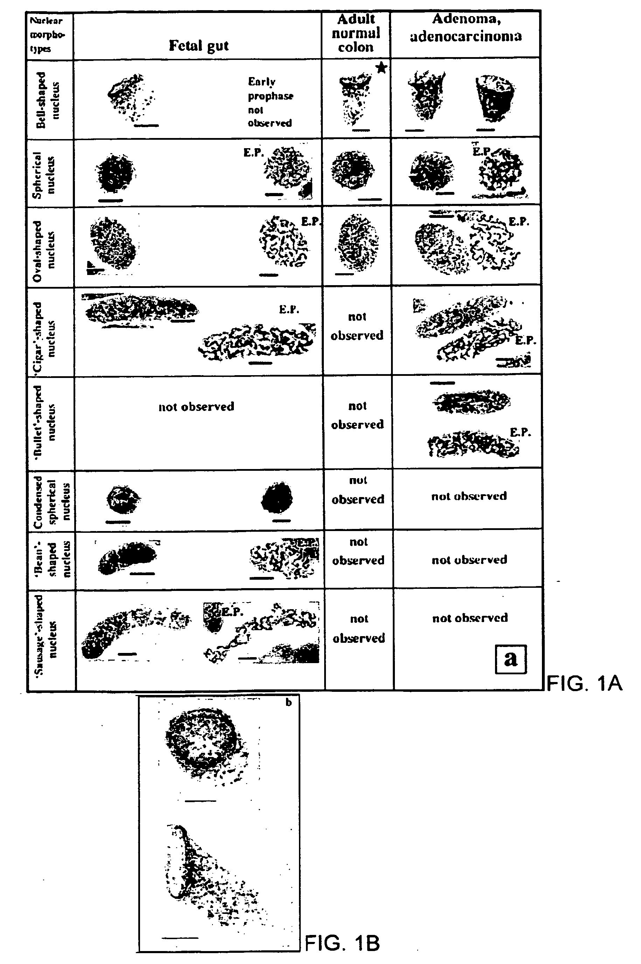

FIG. 1 is a summary of key images. A) Examples of nuclear morphotypes observed in interphase and early prophase (E.P.) cells in human fetal gut, normal colonic mucosa, adenomas and adeno-carcinomas. B) High resolution image (x 1400) of bell-shaped nuclei of fetal gut. Condensed DNA appears to create an annulus that maintains an opening into the hollow bell structure. Scale bar, 5 µm.

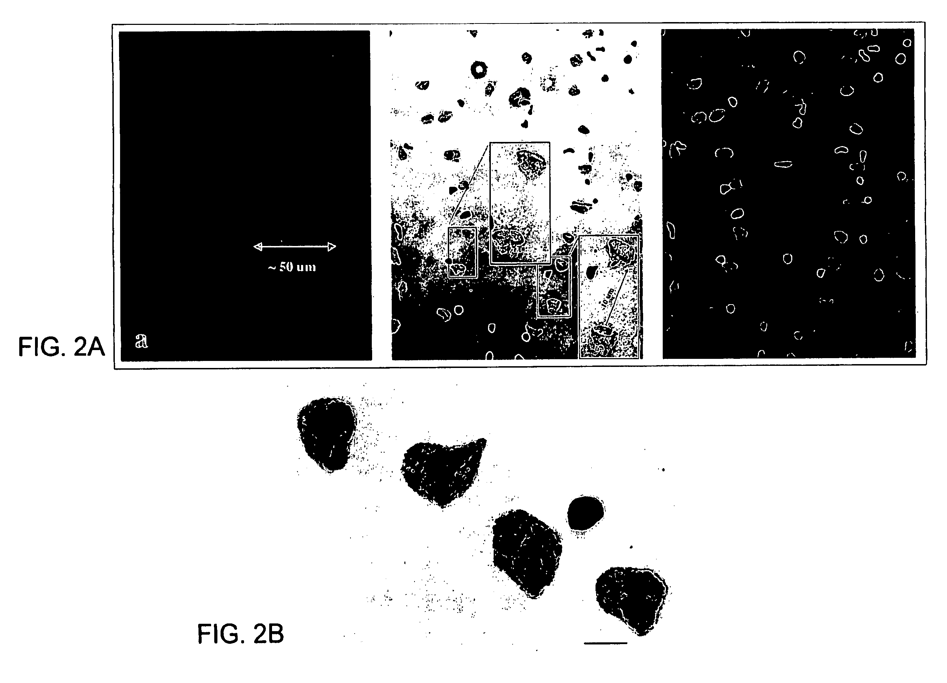

FIGS. 2A and B are images of Embryonic gut, 5-7 weeks. FIG. 2A: Phase-contrast image (left frame) and stained nuclei image (middle) and the merged image (right) show the linear arrays of nuclei within ~50 micron diameter tubular syncytium. FIG. 2B: High resolution image of the nuclei shows hollow bell-shaped structures. The 'head to toe' orientation of the bells is preserved in all embryonic tubes observed but tubes snake backwards and forwards such that parallel tubes may have locally anti-parallel bell-shaped nuclei orientation. Scale bars, 50 µm at low and 5µm at high magnification.

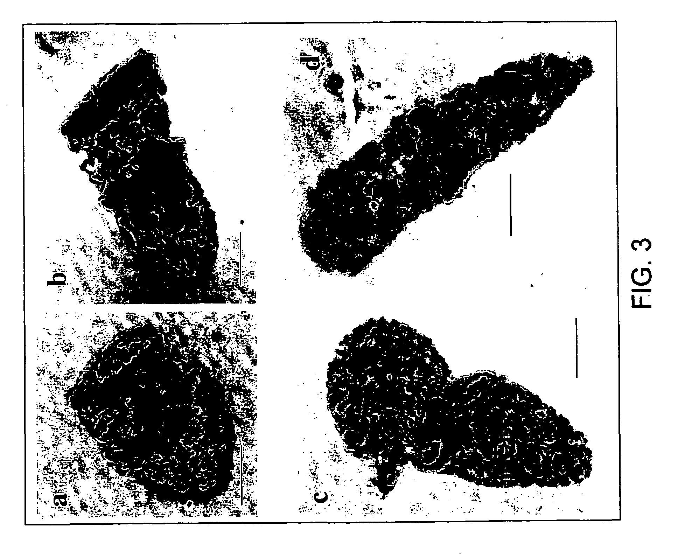

FIGS. 3A-D show images of nuclear fission of bell-shaped nuclei in fetal gut. FIGS. 3A and B: *Symmetrical* nuclear fission. Bell-shaped nuclei emerges from bell-shaped nuclei of similar shape. FIGS. 3C and D: *Asymmetrical* nuclear fission. A spherical nucleus, and a 'cigar'-shaped nuclei emerging from a bell-shaped nucleus. Scale bar, 5 µm.

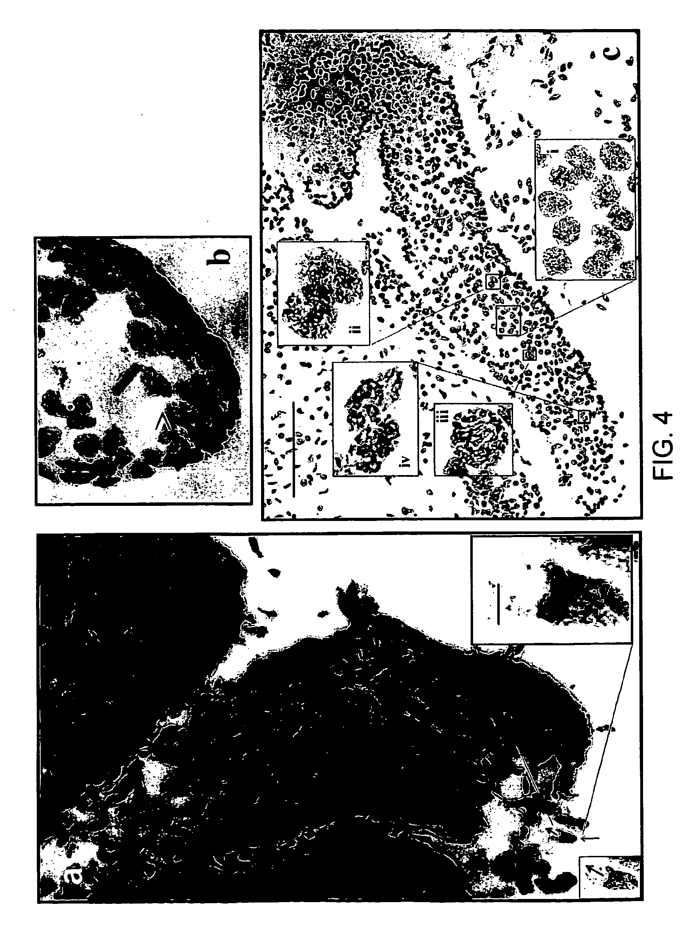

FIGS. 4A-C show images from normal adult colonic crypts. FIG. 4A: Crypts of about 2000 spheroid, spherical or discoid nuclei occasionally (<1/100) contained a recognizable bell-shaped nucleus (arrow) located at the bottom of the crypt. FIG. 4B: Crypt base showing another bell-shaped nucleus. FIG. 4C: Morphotypes of interphase and mitotic nuclei of the walls and luminal surface in a well-spread crypt. The enlarged images show: (i) spherical and ovoid interphase nuclei, (ii, iii) early prophases of spherical- and oval-shaped nuclei, and (iv) an anatelophase nucleus. Scale bars, 100 µm for low and 5 µm for high magnification images.

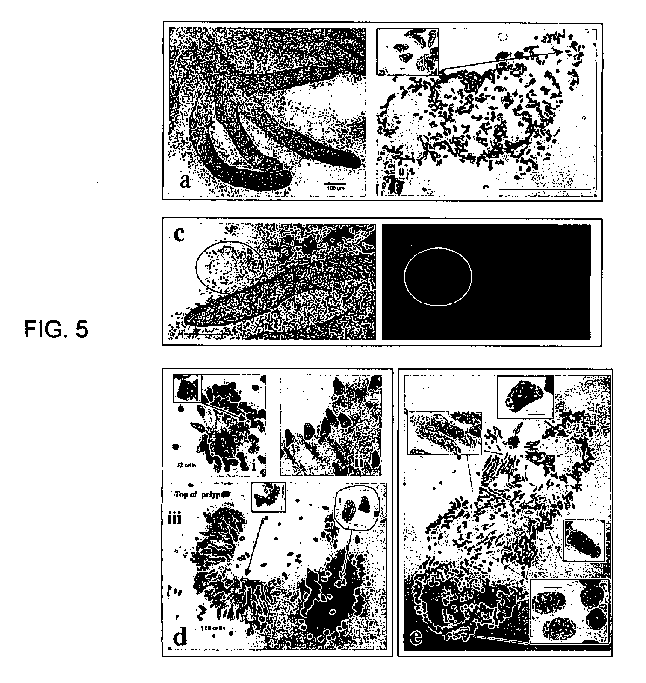

FIGS. 5A-E show images from Adenomas. FIG. 5A: Characteristic large branching crypt of adenomas. FIG. 5B: An irregular crypt-like structure found throughout adenomas. Typically two, but sometimes 1, 4 or even 8, bell-shaped nuclei (insert) appear at the base of these large (>4000 cell) irregular crypt-like structures. FIG. 5C: A cluster of cells of similar nuclear morphotype containing one bell-shaped nucleus. These forms of clusters contain exactly 16, 32, 64, and 128 total cells. Left panel, Feulgen-Giemsa stain. Right panel, phase contrast autofluorescent image. FIG. 5D: Contexts in which bell-shaped nuclei appear in adenomas: (i) Cluster with 31 ovoid nuclei and one bell-shaped nucleus, (ii) Multiple bell-shaped nuclei in shoulder to shoulder arrangement, (iii) Bell-shaped nuclei arranged in a side-by-side pattern (arrow) (iii). Irregular mixture of~250 nuclei of with several bell-shaped nuclei suggestive of nascent crypt bases. FIG. 5E: Irregular crypt-like structure containing apparently clonal patches of cells of 5 different nuclear morphotypes with one bell-shaped nucleus (arrow) at the base. Scale bars, 100 µm (in 'a,b') and 5µm (in 'e').

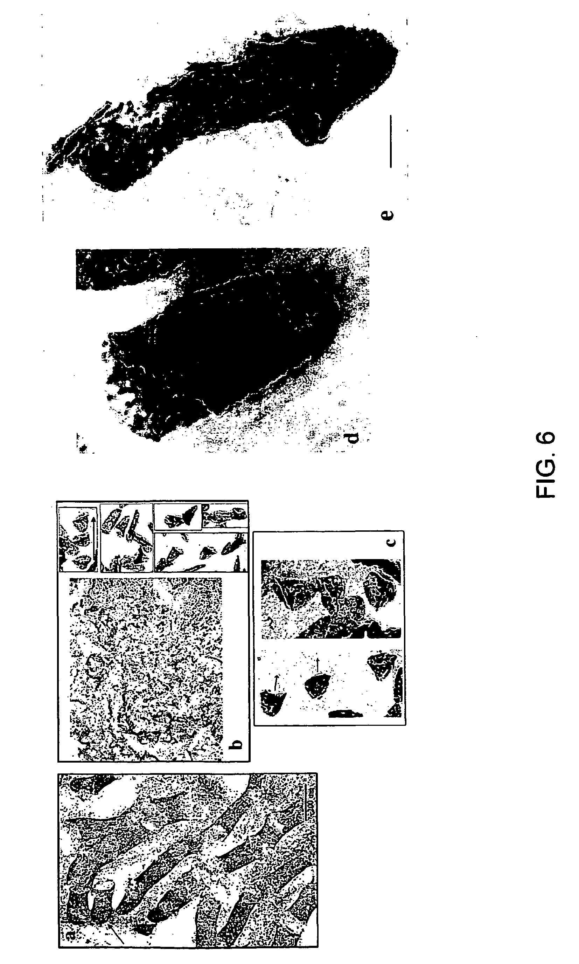

FIGS. 6A-E shows images from adenocarcinomas. FIG. 6A: Very large crypt-like structures (>8000 cells), with branches with frequent break points. The arrow indicates an example of an ~250 cell crypt-like structure found primarily near the surface of the tumor. FIG. 6B: Interior tumor mass with multiple where multiple bell-shaped nuclei (~ 3% of all nuclear morphotypes). FIG. 6C: Bell shaped nuclei in FIG. 6B oriented in head-to-toe syncytial and non-syncytial side-by-side configurations. FIG. 6D: Symmetrical nuclear fission in adenocarcinoma. FIG. 6E: Asymmetrical nuclear fission of a bell creating a cigar-shaped nucleus in adenocarcinoma. Similar structures have been observed in colonic metastases to the liver. Scale bar, 5 µm.

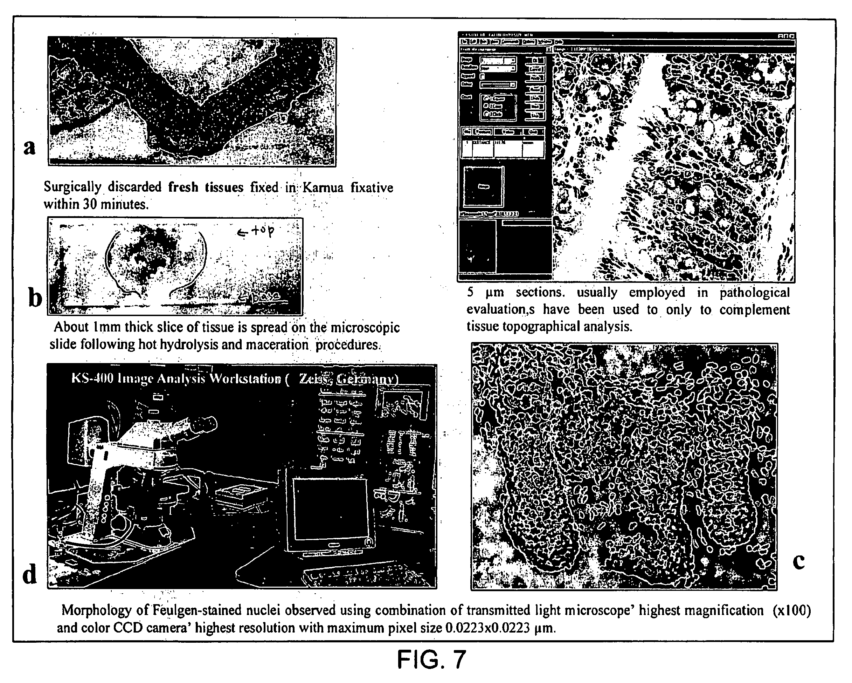

FIGS. 7A-D are illustrations of the stages in quantitative image cytometry in the study of in human tissues and cells. FIG. 7A: Fresh colon surgical discard ready for fixation. FIG. 7B: Microscopic slide preparation showing the result of spreading of 1 mm section through a polyp (positioning of a polyp, 'top- to-bottom' is outlined). FIG. 7C: Cell nuclei spreads (in magenta color) observable for the whole crypts. All of the crypt nuclei are preserved, as compared to 5 µ sections (BrdU staining and H&M staining), shown above. FIG. 7D: Motorized Axioscop microscope- AxioCam color CCD camera- KS 400 software image analysis workstation.

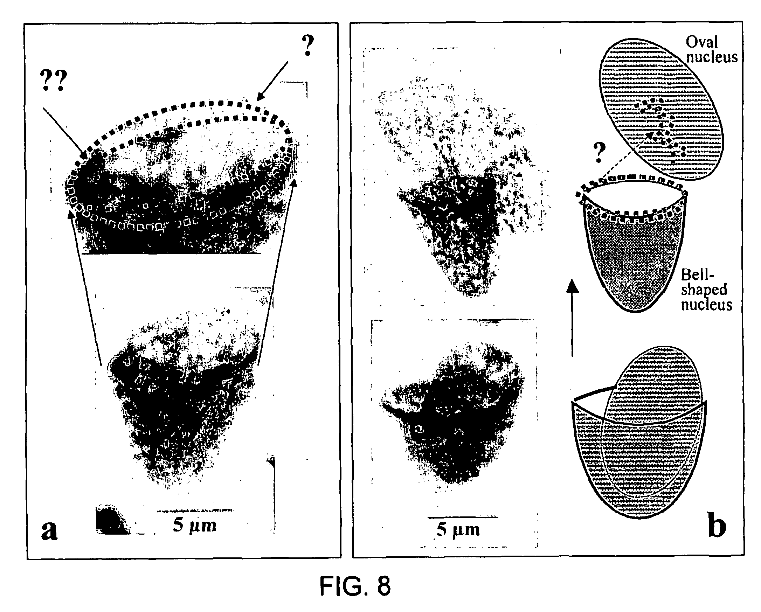

FIGS. 8A and B are illustrations of a 'target of interest' in application of FISH to explore non-dividing and dividing bell-shaped nuclei in tumors. FIG. 8A: Chromatin (stained darker because of higher DNA content per µm²) creates the unique structure resembling prophase chromosomes arranged as two parallel circles. These circles put into drawing illustrate the prediction of that specific chromosomes might be found at this specific site of bell-shaped nuclei. FIG. 8B: Chromatin distribution and specific chromosome positioning changes as imaginary transformation ('bell-to-oval' shaped nuclei here) taking place throughout asymmetrical division of the bell-shaped nuclei.

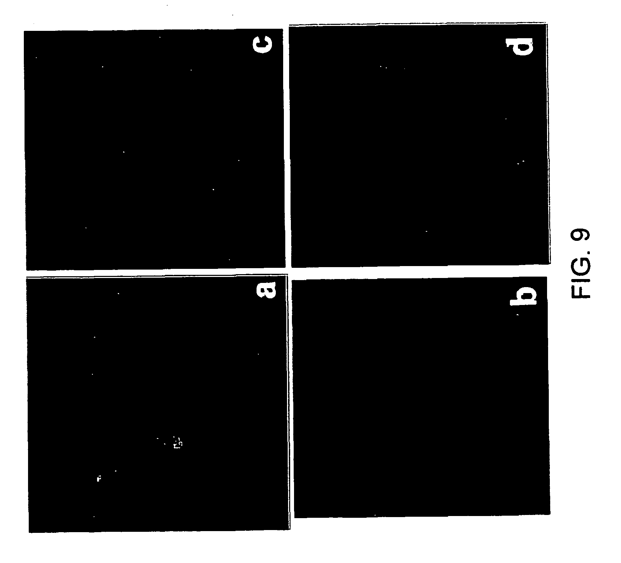

FIGS. 9A-D are images illustrating the results of fluorescent *in situ* hybridization of chromosome 11 in spherical nuclei of TK-6 human cells. FIG. 9A: two pairs of chromosomes in prophase chromosome spreads. FIG. 9B: spherical nuclei DAPI nuclear stain. FIG. 9C: same chromosome pair hybridized with FITC fluorescence probe. FIG. 9D: merged image of DAPI and FITC interphase chromosomes stain. Bar scale, 5 microns.

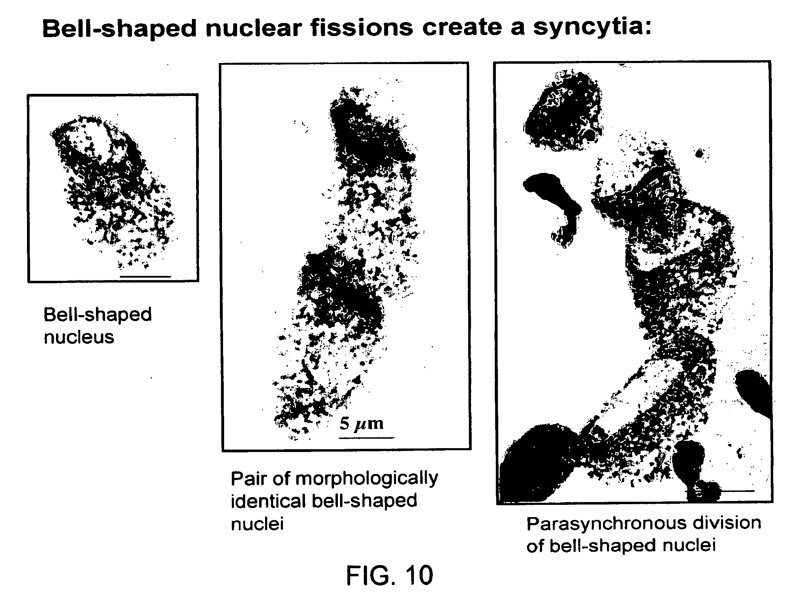

FIG 10 shows images of symmetrical nuclear division of bell-shaped nuclei arranged in syncytia.

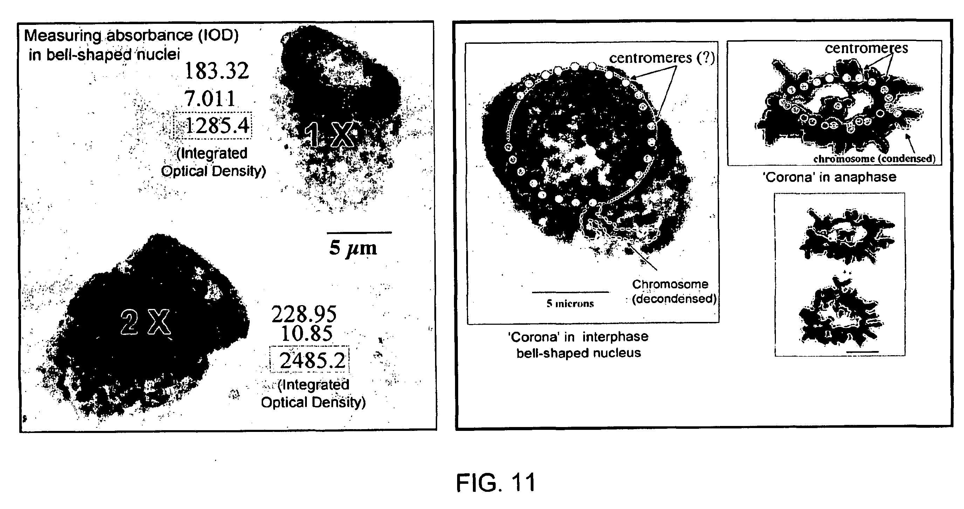

FIG. 11 shows images depicting the localization of DNA in bell-shaped nuclei undergoing nuclear division.

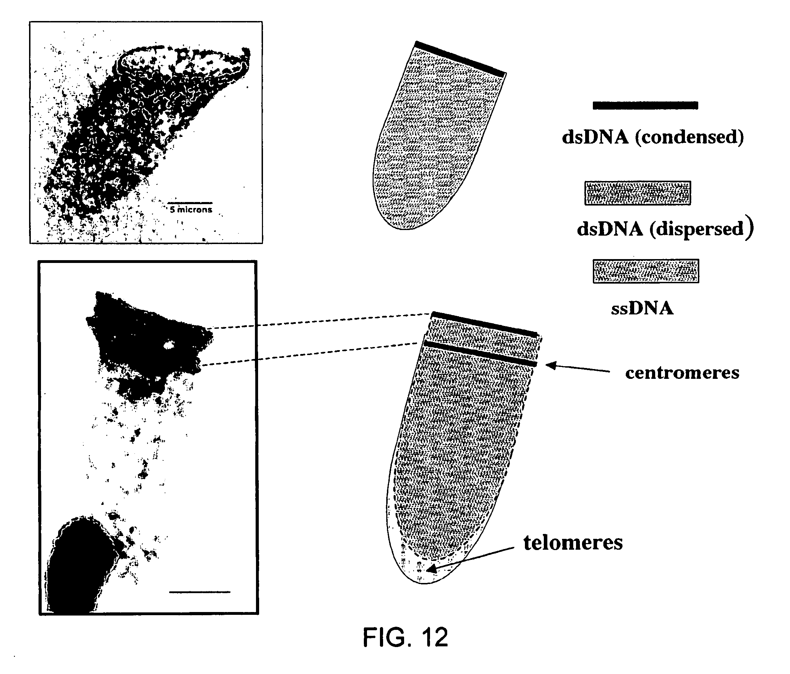

FIG. 12 shows arrangement and composition (ssDNA or dsDNA) of nuclear material during nuclear division of bell-shaped nuclei.

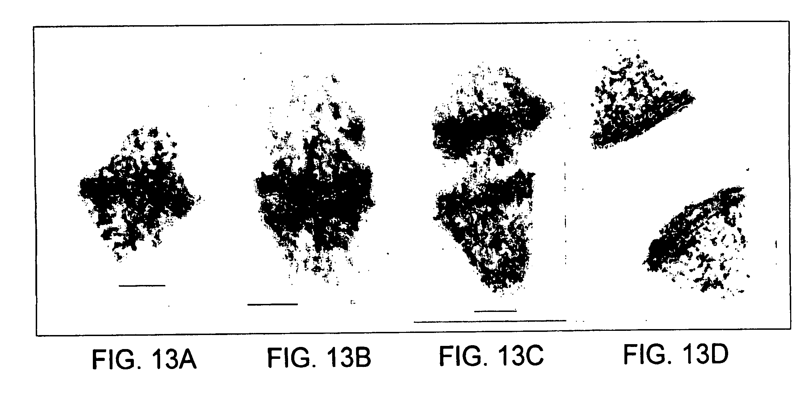

FIGS. 13A-D show images from human fetal preparations depicting a series of previously unrecognized nuclear forms. These forms give rise to the original bell-shaped nuclei. FIG. 13A shows a nucleus with a condensation of ~10% of the total DNA content as a "belt" around the long axis of spherical or slightly oval nuclei. FIG. 13B shows a nucleus in which two condensed nuclear "belts" appear to have separated but are still part of a single nucleus. FIG. 13C shows a pair of nuclei that appear to have arisen by fission of the two-belted nucleus of FIG. 13B. FIG. 13D shows that each syncytium contains a set of bells with a single pair of bells at its linear midpoint with mouths facing as in FIG. 13C. These images show that a series of symmetrical divisions create nuclei pushing away from a central pair.

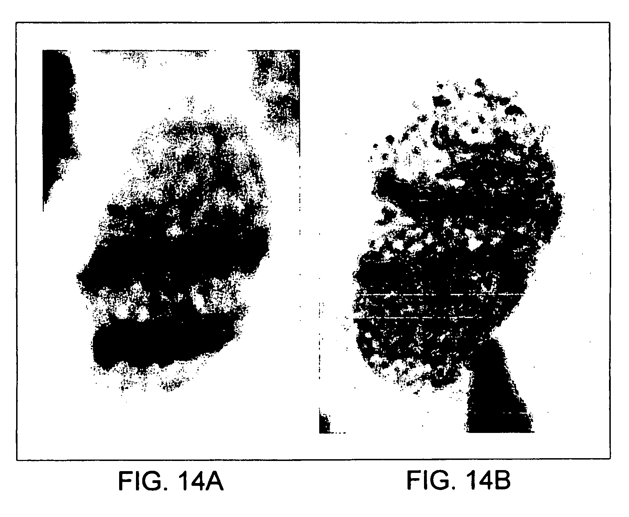

FIGS. 14A and B show nuclear morphotypes in colonic adenomas (FIG. 14A) and adenocarcinomas (FIG. 14B). Morphotypes of carcinogenesis show similar belts- one or two around the long axis of oval nuclei.

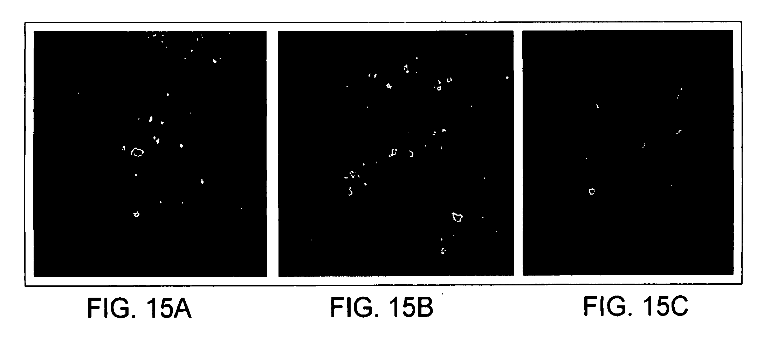

FIGS. 15A-C show FISH staining specific for human centromeres. FIG. 15 shows centromeres (bright) in spherical (FIG. 15A), "cigar"- (FIG. 15B) and bell-(FIG. 15C) shaped nuclei from tissues of human 12 weeks fetal colon.

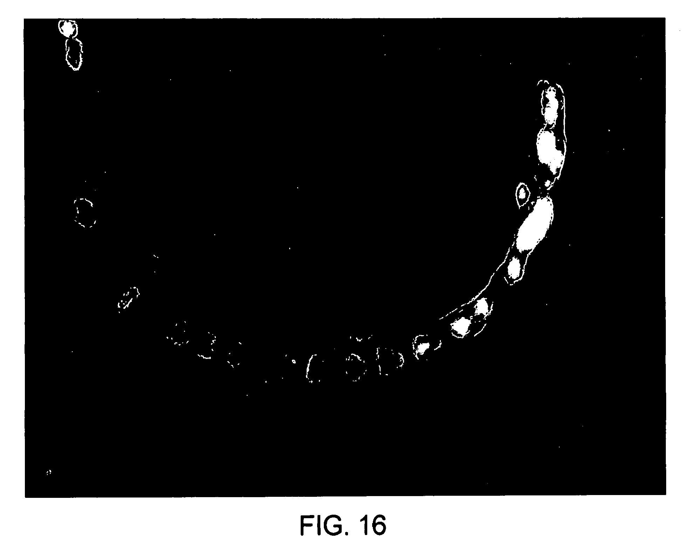

FIG. 16 shows a multi-nucleated syncytium arising from a bell-shaped nucleus in human fetal cardiac muscles, approximately 12 weeks gestation. The particular method involves enzymatic tissue dissociation followed by Acridine orange staining and fluorescence microscopy.

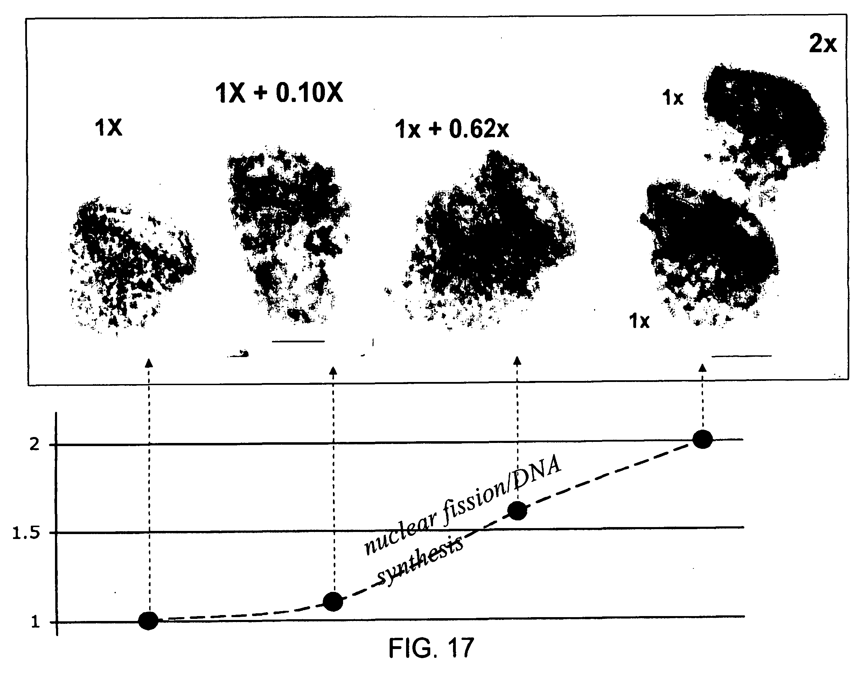

FIG. 17 shows the progression of DNA synthesis in dividing bell-shaped nuclei. The increase in DNA content is indicated above the images and is graphically charted below. The image is captured by high resolution (1000 bp. per pixel) Feulgen DNA image cytometry.

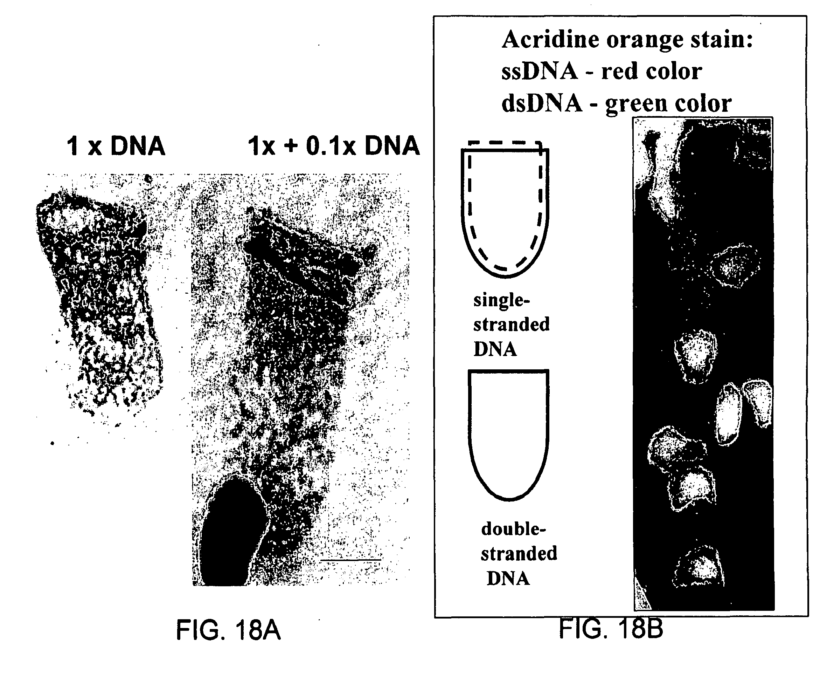

FIGS. 18A-B shows the replicative intermediate configuration of bell-shaped nuclei utilizing ssDNA intermediates. FIG. 18A shows the symmetrical division of bell-shaped nuclei and indicates the corresponding increase in DNA content. FIG. 18B shows the Acridine orange stain of a bell-shaped nuclei. Red fluorescence of the Acridine orange stain indicates the presence of ssDNA intermediates. Double-stranded DNA in adjacent cells is indicated by green fluorescence of the Acridine orange stain.

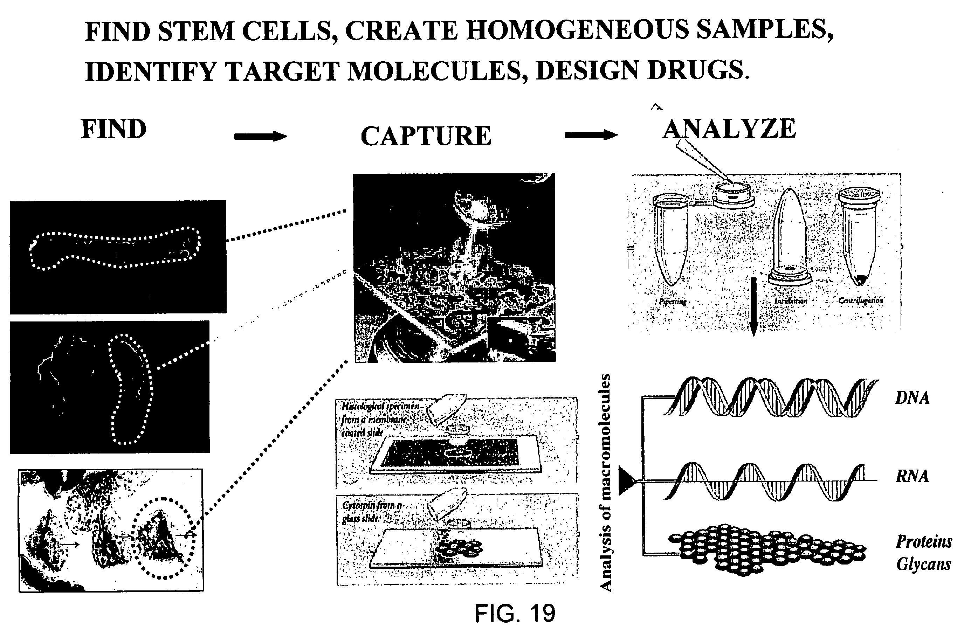

FIG. 19 shows a flow diagram of the method to locate tumor stem cells, create homogeneous samples, and identify target macromolecules specific to said stem cells for intelligent therapeutic design.

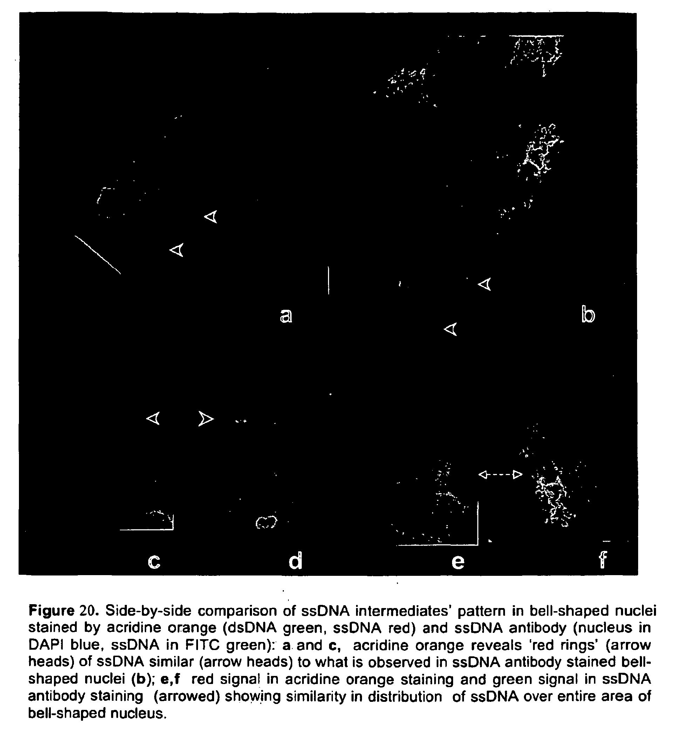

FIGS. 20A-F show side-by-side comparison of ssDNA intermediates' pattern in bell-shaped nuclei stained by acridine orange and ssDNA antibody.

### DETAILED DESCRIPTION OF THE INVENTION

The presented invention relates to the discovery that tumor stem cells, e.g. cells that divide leading to tumors, are characterized by bell-shaped nuclei and undergo a unique form of replication. Bell-shaped nuclei, are rarely found in adult tissues, or found in greatly reduced numbers, except for tumor tissues, undergo periods of time where the genome is represented as single-stranded DNA (ssDNA). Bell-shaped nuclei divide both symmetrically and asymmetrically by non-mitotic fission processes in colonic and pancreatic human tumors (Gostjeva et al., Cancer Genetics and Cytogenetics, 164:16-24 (2006); Gostjeva et al., Stem Cell Reviews, 1: 243-252 (2005)). These bell-shaped nuclei appear in great numbers both in 5-7 week embryonic hindgut where they are encased in tubular syncytia, and comprise, for example, 30% of all nuclei and tumor tissues where they abound in "undifferentiated" niches. They possess several stem cell-like qualities, particularly the "shibboleth" of asymmetric division and a nuclear fission frequency consistent with growth rates of human colonic preneoplastic and neoplastic tissue (Herrero-Jimenez, P., et al., Mutat.Res., 400:553-78 (1998), Herrero-Jiminez, P., et al., Mut. Res. 447:73-116 (2000); Gostjeva et al., Cancer Genetics and Cytogenetics, 164:16-24 (2006); Gostjeva et al., Stem Cell Reviews, 1: 243-252 (2005)) These previously unrecognized nuclear forms are both the source of tumor generation and differentiation and thus targets for cancer therapeutic strategies. The observation that these bell-shaped nuclei undergo a stage where the genome is substantially represented as ssDNA allows for their targeting and destruction.

### Methods for Identifying Anti-Tumorigenic Agents

The unexpected observation described herein is that tumor stem cell replication involves a replicative intermediate configuration wherein the bell-shaped nuclei commences separation into two nuclei, and that during nucleic separation, a substantial fraction of the tumor stem cell genome is first segregated into ssDNA. During this replicative intermediate configuration, large amounts of ssDNA are present in the nuclei and in cell that can be detected using routine methods. This observation underlies the basis for the methods of screening and identifying efficacious anti-tumorigenic agents described herein that evaluate the agents' usefulness at inhibiting/preventing the proliferation of bell-shaped nuclei. Further, tumor stem cell-specific molecules can also be present in the cell during this replicative intermediate configuration and can also serve as the basis of assay methods. Using methods described herein and in US Patent Application 2006/0063144 (US patent 7, 427, 502) and PCT/US06/047136 International patent publication no. WO 2007/067795 one can visualize bell-shaped nuclei containing ssDNA or ssDNA alone in a solid tumor mass, both before and after contact or treatment to a candidate anti-tumorigenic agent, and compare the relative density of cells with bell-shaped nuclei (metakaryotes) either before and after treatment with the candidate/test agent, or to other tumor tissues that have not been contacted/treated with the agent. A decrease in the relative density, e.g., number of bell-shaped nuclei containing ssDNA or ssDNA alone relative to number of cells or relative to weight of the tumor mass, would indicate that the agent is effective in reducing or preventing the proliferation of tumor stem cells.

For example, a tumor sample, e.g., obtained from a patient or from a solid tumor formed as a xenograft into a host organism, would be exposed to an anti-tumorigenic agent, e.g., an agent that targets ssDNA and degrades ssDNA, prevents annealing of ssDNA, or prevents replication of a ssDNA template, etc., based on the ssDNA intermediate configuration present when bell-shaped nuclei divide asymmetrically, after the relative density of bell-shaped nuclei containing ssDNA had been determined in the whole or part of the tumor sample. After exposure to the agent, the relative density of bell-shaped nuclei can again be determined. If the density of containing ssDNA is decreased or absent entirely, then it would be reasonable to believe that the candidate agent would be an effective anti-tumorigenic agent.

More specifically, described herein is a method for identifying an agent that inhibits tumor growth, comprising contacting a tumor sample in a subject or host animal with a candidate agent, wherein the tumor sample comprises tumor stem cells containing bell-shaped nuclei containing ssDNA or ssDNA alone, and wherein some or all of the bell-shaped nuclei are in a replicative intermediate configuration; maintaining the sample in contact with the candidate agent under conditions suitable for the agent to interact with ssDNA; determining the presence of, absence of, or number of bell-shaped nuclei containing ssDNA or ssDNA alone in the tumor sample after the contact; and comparing the presence of, absence of, or number of bell-shaped nuclei containing ssDNA or ssDNA alone in the sample after contact with the candidate agent to the presence of, absence of, or number of bell-shaped nuclei containing ssDNA or ssDNA alone in a control sample. Determination of a reduction in or absence of, in the number of bell-shaped nuclei containing ssDNA, or reduction in or absence of ssDNA in the sample after contact with the candidate agent indicates that the agent inhibits tumor growth.

Alternatively, the presence of, absence of, or number of bell-shaped nuclei can be determined by detecting the presence of one, or more tumor stem cell-specific molecules in, or in close proximity of, the replicative intermediate configuration. Moreover, the methods described can be used to detect such macromolecules specific to the replicative intermediate configuration of bell-shaped nuclei. Because the bell-shaped nuclei of tumor stem cells represent an event not found in other human cells, the identification of these molecules facilitates the identification of targets for therapeutic drug design. Further, because a large fraction of the DNA in tumor stem cells is single-stranded during replication, stem cells in the process of symmetric division can be isolated for identification of macromolecules by those skilled in the art. Examples of potential macromolecules that may be involved with the replicative intermediate configuration include: ssDNA-binding proteins such as heterogeneous nuclear ribonuclear proteins; structural maintenance of chromosomes (SMC) proteins involved in physical movement and separation of the ssDNA; novel centromeric proteins; enzymes involved in DNA repair; novel polymerases; and an array of yet unidentified proteins.

Suitable heterogeneous animal model could be created by injecting the animal with a cancer xenograft. The xenograft would then be allowed to mature into solid tumor, which could then be excised. The presence of cells with bell-shaped nuclei, metakaryotes or syncytia with bell-shaped nuclei could be confirmed as described herein and in PCT/US06/047136. The animal and/or tumor could then be exposed to agents known to prevent reannealing of ssDNA, bind, modify or degrade ssDNA such as, for example, alkylating agents, ssDNA nucleases, or other enzymes or proteins, including agents such as, for example, antibodies, oligonucleotides, ribozymes, riboproteins, or fusion proteins that target ssDNA. Some non-limiting examples include carmustine, lomustine, cyclophosphamide, chlorambucil, ifosfamide.

Unlike other tumor models that look for activity of agents based on the shrinkage of the growing tumor or the survival patterns of the xenotransplanted animal relative to control, this strategy involves assessing the growing xenotransplant for frequency of ssDNA staining metakaryotic cells. Agents of interest are agents that significantly reduce the number of, for example, acridine orange-staining metakaryotes relative to a control group (e.g., and untreated tumor sample or a portion or whole of the same tumor sample prior to exposure to the agent). It is reasonable to believe that agents/compounds that interfere with the ssDNA replicative intermediate of dividing tumor cells are expected to target the metakaryotic process, and thus be effective agents for the treatment, not of tumor bulk, but of the residual surfacing tumor that is not treated with standard agents that target the rapidly dividing mitotic tumor cells. One of skill in the art would need to only look for changes occurring in the quantity or quality of the ssDNA of bell-shaped nuclei, ssDNA within the metakaryote, as well as any morphological changes occurring in the bell-shaped cell and the tumor itself, to determine whether the candidate agent (or agent of interest) would be a viable candidate for further evaluation. In particular, changes in tumor stem cell morphology, changes in the amount of ssDNA within the metakaryote, or the morphology of the tumor itself, can be indicative that the candidate agent is effective in inhibiting tumor growth. Such changes would be manifested by a decrease in, or absence of, tumor stem cells.

### The "Stem Cell Target" in Cancer Therapeutics

As a result of the data presented herein, methods are now available to deliver agents to target and destroy tumor stem cells without or with minimal damage to cells in their close environment. Methods are available for treating preneoplasias, neoplasias (carcinoma) or other pathologies in an individual, by administering an ssDNA-specific agent, or an agent that targets a tumor stem cell-specific molecule.. The term, "treatment" as used herein, can refer to ameliorating symptoms associated with the carcinoma or pathology; to reducing, preventing or delaying metastasis of the carcinoma; to reducing the number, volume, and/or size of one or more tumors; and/or to lessening the severity, duration or frequency of symptoms of the carcinoma or pathology. A "ssDNA-specific therapeutic agent," as used herein, refers to an agent that targets the ssDNA of tumor stem cells for destruction by prevention of the formation of ssDNA, interference with the replication of ssDNA or interference of copying of ssDNA (e.g., a chemotherapeutic agent), or otherwise treats the carcinoma, or reduces or eliminates the effects of tumor(s) or pathologies on the individual. As described herein, ssDNA is important in tumor stem cell maintenance, therefore destruction of ssDNA resulting in the prevention of the formation of ssDNA, interference with the replication of ssDNA or interference of copying of ssDNA, will inhibit tumor stem cell replication and thereby treat the carcinoma or pathology. The methods of treatment described are used in conjunction with one or more of surgery, hormone ablation therapy, radiotherapy or chemotherapy. The chemotherapeutic, hormonal and/or radiotherapeutic agent and compound may be administered simultaneously, separately or sequentially.

Chemical or enzymatic agents that target and alter ssDNA *(e.g.,* alkylating reagents, single-strand-specific nucleases, agents that target replication machinery, etc.) can be utilized to target the nuclear material of tumor stem cells for destruction, as the modified ssDNA would be unable to undergo further replication back into double-stranded DNA (dsDNA). Specific tumor cell inhibition is achieved through the use of agents that specifically target ssDNA, as ssDNA is significantly more abundant in the heteromorphic nuclear morphotypes of dividing tumor stem cells than in adjacent cells. For example, any agent that prevents replication of ssDNA, *e.g.,* a molecule that hybridizes to DNA, but is incapable of being extended, *e.g.,* a modified oligonucleotide or nucleic acid derivative, *e.g.,* a nucleic acid lacking the γ-phosphate necessary for extension or a peptide nucleic acid. It is described a method of therapeutically treating a patient having a cancerous tumor by specifically targeting the ssDNA of the tumor stem cells within that tumor, wherein the method comprises administering to the patient a therapeutically effective amount of an alkylating agent, a DNA nuclease, DNA-binding polypeptide, an oligonucleotide or a small organic molecule that binds specifically to the single-stranded DNA and destroys the tumor stem cell, thereby resulting in the effective therapeutic treatment of the tumor. Optionally, the polypeptide, oligonucleotide or oligopeptide may be conjugated to a growth inhibitory agent or cytotoxic agent, a radioactive isotope, a nucleolytic enzyme, or the like. Methods are known in the art for delivering agents to cells or tumor tissue in a patient, where such agents would destroy or prevent replication of the ssDNA genome, and thereby prevent proliferation of the tumor stem cells.

An agent is delivered in a tumor stem cell-specific manner, utilizing an agent or moiety that specifically binds to ssDNA. An agent that "specifically binds" to ssDNA, as the term is used herein, is an agent that preferentially or selectively binds to ssDNA and not to dsDNA. While certain degree of non-specific interaction may occur between the agent that specifically binds ssDNA and double-stranded DNA, nevertheless, specific binding, may be distinguished as mediated through specific recognition of ssDNA, in whole or part. Typically, specific binding will be favored by the relative abundance of ssDNA in tumor stem cells.

Another method specifically targets the ssDNA of tumor stem cells. The ssDNA may be free in the nucleus or may be bound into nucleoprotein complexes. The ssDNA exists in unique structural conformations that those skilled in the art will be able to utilize for specific targeting. For example, thermodynamics favors the formation of cruciform structures and hairpin loops. Antibodies can be utilized to specifically target these structures. An "antibody" is an immunoglobulin molecule obtained by *in vitro* or *in vivo* generation of the humoral response, and includes both polyclonal and monoclonal antibodies. The term also includes genetically engineered forms such as chimeric antibodies (e.g., humanized murine antibodies), heteroconjugate antibodies (e.g., bispecific antibodies), and recombinant single chain Fv fragments (scFv). The term "antibody" also includes antigen binding fragments of antibodies, such as Fab', F(ab')₂, Fab, Fv, rIgG, and, inverted IgG, as well as the variable heavy and variable light chain domains. An antibody immunologically reactive with ssDNA can be generated *in vivo* or by recombinant methods such as selection of libraries of recombinant antibodies in phage or similar vectors. See, e.g., Huse et al. (1989) Science 246:1275-1281; and Ward, et al. (1989) Nature 341:544-546; and Vaughan et al. (1996) Nature Biotechnology, 14:309-314. An "antigen binding fragment" includes any portion of an antibody that binds to ssDNA. An antigen binding fragment may be, for example, a polypeptide including a CDR region, or other fragment of an immunoglobulin molecule which retains the affinity and specificity ssDNA. A number of ssDNA antibodies are commercially available. In a specific example, one skilled in the art could identify the single-chain Fv fragment of the ssDNA-binding antibody, which is the minimum antibody form still retaining specificity and monovalent binding affinity of the full-size parent. The single-chain Fv fragment is expressed as a single gene and could be fused to a nuclear localization signal to direct the antibody fragment to the nucleus. It is reasonable to believe that the single-chain Fv fragment could be introduced and expressed within a tumor cell group by means of mircoinjection or viral-mediated gene therapy. Once bound to the abundant secondary structures of ssDNA, the antibody fragment would block replication and division of the tumor stem cells.

One skilled in the are could express and purify polypeptides of known, specific ssDNA binding domains in vitro utilizing well-known methods. For example, the ssDNA-specific domains of polyADP Ribose Polymerase (PARP) or the heterogeneous nuclear ribonuclear family of proteins could be expressed via a baculovirus or other eukaryotic protein expression system. These agents could be further fused to in frame to cationic cell-penetrating peptide sequences to facilitate rapid cellular uptake of the biologically-active peptide. Alternatively, microinjection could be utilized to introduce the polypeptides into the cell.

Cationic phosphoramidate oligonucleotides are introduced into the tumor stem cells to efficiently target single-stranded DNA. One skilled in the art could manufacture these agents is such a manner that they would thoroughly and randomly target multiple regions of ssDNA These agents bind with high affinity to ssDNA and readily pass into target cells without vectorization or chemical transfection.

ssDNA can be specifically targeted through chemical agents. These agents react preferentially with ssDNA nucleotide bases in a sequence- or secondary structure-dependent manner. Examples include Actinomycin D, potassium permanganate, bromoacetaldehyde, chloroacetaldehyde, diethyl pyrocarbonate, and osmium tetroxide, as set forth in Table 1 below. Although some of these substances are toxic at high levels, it is reasonable to believe that the agents can be introduced into the tumor in a localized and dose-selective manner, thereby minimizing toxicity. Similarly, although some chemical agents bind both ssDNA and double-stranded DNA, the abundant level of ssDNA is tumor stem cells facilitates an extremely low and more effective therapeutic dose.

**TABLE 1: Chemical agents that bind ssDNA.**

| **Chemical Agent** | **Mechanism** |
|---|---|
| Actinomycin D | Extensively studied anti-tumor agent. Binds to hairpin DNA structures abundant in ssDNA. |
| Bromoacetaldehyde | Reacts at the base-pairing positions of adenines and cytosines. Preferentially reacts with bases in single-stranded loops and cruciforms. |
| Chloroacetaldehyde | A metabolite of vinyl chloride that readily interacts with ssDNA to predominantly form etheno lesions. |
| Diethyl pyrocarbonate | Carboxyethylates purines at the N-7 position, which opens the imidazole ring. Substantially reactivity toward single-stranded regions of DNA. |
| Osmium tetroxide | Adds to the C-5, C-6 double bond of pyrimidines in the presence of pyridine to form osmate esters. Substantially more reactive to ssDNA than double-stranded DNA. |
| Potassium permanganate | Pyrimidine-specific and single-strand specific. Modifies bases via oxidation. |

The ssDNA therapeutic agent comprises an active agent component/moiety and a targeting agent component/moiety. The targeting agent component is or comprises an agent that specifically binds to ssDNA, as described above. The targeting agent component is linked to the active agent component. For example, they can be covalently bonded directly to one another. Where the two are directly bonded to one another by a covalent bond, the bond may be formed by forming a suitable covalent linkage through an active group on each moiety. For instance, an acid group on one compound may be condensed with an amine, an acid or an alcohol on the other to form the corresponding amide, anhydride or ester, respectively. In addition to carboxylic acid groups, amine groups, and hydroxyl groups, other suitable active groups for forming linkages between a targeting agent component and an active agent component include sulfonyl groups, sulfhydryl groups, and the haloic acid and acid anhydride derivatives of carboxylic acids.

The therapeutic agent can comprise two, or more moieties or components, typically a targeting agent moiety with one or more active agent moieties. Linkers can be used to link an active agent to a targeting agent component, wherein the targeting agent specifically interacts with ssDNA, or a tumor stem cell-specific molecule, thereby delivering the active agent to the replicative intermediate configuration, and inhibiting further replication of the bell-shaped nuclei.

The active agent component, which is linked to the targeting agent component, can be or comprise any agent that achieves the desired therapeutic result, including agents such as: a radionuclide (e.g., I125, 123, 124, 131 or other radioactive agent); a chemotherapeutic agent (e.g., an antibiotic, antiviral or antifungal); an immune stimulatory agent (e.g., a cytokine); an anti-neoplastic agent: an anti-inflammatory agent; a pro-apoptotic agent (e.g., peptides); a toxin (e.g., ricin, enterotoxin, LPS); an antibiotic; a hormone; a protein (e.g., a surfactant protein, a clotting protein); a lytic agent; a small molecule (e.g., inorganic small molecules, organic small molecules, derivatives of small molecules, composite small molecules); nanoparticles (e.g, lipid or non-lipid based formulations); lipids; lipoproteins; lipopeptides; liposomes; lipid derivatives; a natural ligand; an altered protein (e.g., albumin or other blood carrier protein-based delivery system, modified to increase affinity for ssDNA or a derivative of annexin A1, orosomucoid); a nucleolytic enzyme; an agent that inhibits growth, migration or of the tumor stem cell; a gene or nucleic acid (e.g., an antisense oligonucleotide); viral or non-viral gene delivery vectors or systems; or a prodrug or promolecule. One skilled in the art will be familiar with the design and application of the active agent.

For example, a radionuclide or other radioactive agent can be used as the active agent component. The targeting agent component delivers the radioactive agent in a tumor-specific manner, allowing local radiation damage and resulting in radiation-induced apoptosis and necrosis throughout the tumor including in tumor cells, stromal calls, and endothelial cells of the tumor.

Chemotherapeutic agents for neoplastic diseases can be used as the active agent component. Representative agents include alkylating agents (nitrogen mustards, ethylenimines, alkyl sulfonates, nitrosoureas, and triazenes) and other similar agents. For example, the chemotherapeutic agent can be acytotoxic or cytostatic drugs. Chemotherapeutics may also include those which have other effects on cells such as reversal of the stem cell state to a differentiated state or those which inhibit cell replication. Examples of known cytotoxic agents useful are listed in, for example, in Goodman et al., "The Pharmacological Basis of Therapeutics," Sixth Edition, A. G. Gilman et al, eds./Macmillan Publishing Co. New York, 1980. Some are set forth in Table 2 below.

**TABLE 2: Chemotherapeutic agents.**

| **Nitrogen Mustard Analogues** | **Alkyl Sulfonates** | **Ethylenimines** | **Nitrosourea** | **Triazines** |
|---|---|---|---|---|
| Cyclophosphamide | Busulfan | Thiotepa | Carmustine | Procarbazine |
| Chlorambucil | Treosulfan | Triaziquone | Lomustine | Dacarbazine |
| Melphalan | Manosulfan | Carboquone | Semustine | |
| Chlormethine | Melphalan | | Streptozocin | |
| Ifosfamide | | | Fotemustine | |
| Trofosfamide | | | Nimustine | |
| Prednimustine | | | Ranimustine | |
| Mechlorethamine | | | | |

Additional cytotoxic agents include vinca alkaloids, such as vinblastine and vincristine; enzymes, such as L-asparaginase; platinum coordination complexes, such as cisplatin, carboplatin, and oxaliplatin; substituted urea, such as hydroxyurea; and methyl hydrazine derivatives, such as procarbazine. As ssDNA presents a greater number of potential alkylation targets than double-stranded DNA, the methods described herein represent a means of improving the efficacy of conventional chemotherapeutic DNA alkylating agents. Notable drawbacks of conventional chemotherapeutic regimes include non-specificity and adverse side effects. With the selective targeting of ssDNA in a limited population of vulnerable tumor cells it is reasonable to believe that a dosing regime can be recalculated for improved efficacy.

Most of the chemotherapeutic agents currently in use in treating cancer possess functional groups that are amenable to chemical crosslinking directly with an amine or carboxyl group of a targeting agent component. For example, free amino groups are available on cis-platin, while free carboxylic acid groups are available on melphalan and chlorambucil. These functional groups, that is free amino and carboxylic acids, are targets for a variety of homobifunctional and heterobifunctional chemical crosslinking agents which can crosslink these drugs directly to a free amino group.

The present application specifically contemplates methods where the targeting agent component and/or the active agent component comprises a chelate moiety for chelating a metal, e.g., a chelator for a radiometal or paramagnetic ion. In preferred embodiments, the a chelator is a chelator for a radionuclide. Radionuclides useful within the present invention include gamma-emitters, positron-emitters, Auger electron-emitters, X-ray emitters and fluorescence-emitters, with beta- or alpha-emitters preferred for therapeutic use. Examples of radionuclides useful as toxins in radiation therapy include: ³²P, ³³P, ⁴³K, ⁴⁷Sc, ⁵²Fe, ⁵⁷Co, ⁶⁴Cu, ⁶⁷Ga, ⁶⁷Cu, ⁶⁸Ga, ⁷¹Ge, ⁷⁵Br, ⁷⁶Br, ⁷⁷Br, ⁷⁷As, ⁷⁷Br, ⁸¹Rb/^{81M}Kr, ^{87M}Sr, ⁹⁰Y, ⁹⁷Ru, ⁹⁹Tc, ¹⁰⁰Pd, ¹⁰¹Rh, ¹⁰³Pb, ¹⁰⁵Rh, ¹⁰⁹Pd, ¹¹¹Ag, ¹¹¹In, ¹¹³In, ¹¹⁹Sb ¹²¹Sn, ¹²³I, ¹²⁵I, ¹²⁷Cs, ¹²⁸Ba, ¹²⁹Cs, ¹³¹I, ¹³¹Cs, ¹⁴³Pr, ¹⁵³Sm, ¹⁶¹Tb, ¹⁶⁶Ho, ¹⁶⁹Eu, ¹⁷⁷Lu, ¹⁸⁶Re, ¹⁸⁸Re, ¹⁸⁹Re, ¹⁹¹Os, ¹⁹³Pt, ¹⁹⁴Ir, ¹⁹⁷Hg, ¹⁹⁹Au, ²⁰³Pb, ²¹¹At, ²¹²Pb, ²¹²Bi and ²¹³Bi. Preferred therapeutic radionuclides include ¹⁸⁸Re, ¹⁸⁶Re, ²⁰³Pb, ²¹²Pb, ²¹²Bi, ¹⁰⁹Pd, ⁶⁴Cu, ⁶⁷Cu, ⁹⁰Y, ¹²⁵I, ¹³¹I, ⁷⁷Br, ²¹¹At, ⁹⁷Ru, ¹⁰⁵Rh, ¹⁹⁸Au and ¹⁹⁹Ag, ¹⁶⁶Ho or ¹⁷⁷Lu. Conditions under which a chelator will coordinate a metal are described, for example, by Gansow et al., U.S. Pat. Nos. 4,831,175, 4,454,106 and 4,472,509.

^{99m}Tc is a particularly attractive radioisotope for this application, as it is readily available to all nuclear medicine departments, is inexpensive, gives minimal patient radiation doses, and can be easily tethered so DNA oligonucleotides via the hydrazino nicotinamide moity. (See e.g., Hnatowich, et al., J. of Nuclear Med., 36:2306-2314 (1995)). A selection of random DNA oligomers can be labeled with technetium and will bind to the accessible and abundant corresponding sequences in the ssDNA of tumor stem cells. ^{99m}Tc has a half-life of six hours which means that rapid targeting of a technetium-labeled oligomer is desirable. Accordingly, the therapeutic agent includes a chelating agents for technium.

The therapeutic agent can also comprise radiosensitizing agents, e.g., a moiety that increase the sensitivity of cells to radiation. Examples of radiosensitizing agents include nitroimidazoles, metronidazole and misonidazole (See e.g., DeVita, V. T. Jr. in Harrison's Principles of Internal Medicine, p.68, McGraw-Hill Book Co., N.Y. 1983 . The ssDNA therapeutic agent that comprises a radiosensitizing agent as the active moiety is administered and localizes at the metastasized cell. Upon exposure of the individual to radiation, the radiosensitizing agent is "excited" and causes the death of the cell.

There are a wide range of moieties which can serve as chelating ligands and which can be derivatized as part of the ssDNA therapeutic agent. For instance, the chelating ligand can be a derivative of 1,4,7, 10-tetraazacyclododecanetetraacetic acid (DOTA), ethylenediaminetetraacetic acid (EDTA), diethylenetriaminepentaacetic acid (DTPA) and 1-p-Isothiocyanato-benzyl-methyldiethylenetriaminepentaacetic acid (ITC-MX). These chelators typically have groups on the side chain by which the chelator can be used for attachment to a targeting agent component. Such groups include, e.g., benzylisothiocyanate, by which the DOTA, DTPA or EDTA can be coupled to, e.g., an amine group of the inhibitor.

Alternatively, the application includes use of ssDNA-specific nucleolytic enzymes to destroy the ssDNA of tumor cells. For example, DNAse VI, S 1 nuclease, RAD2, or any other human endonuclease specific for ssDNA could be ectopically expressed within a tumor mass via viral-mediated gene expression or microinjection. One trained in the art would be familiar with these well-known methods. Because tumor stem cells harbor substantially elevated levels of ssDNA compared to neighboring cells, the nucleases will have an enhanced cytotoxic effect on tumor stem cells. One skilled in the art could engineer catalytic RNA molecules (ribozymes) to subject the unhindered and unprotected nucleotide backbone of ssDNA to nucleophilic attack. For example, specific cleavage of single-stranded DNA molecules under physiologic conditions can be achieved via a catalytic RNA molecule having a deoxyribonuclease activity. A specific example is a ribonucleotide polymer that has a 5' terminal nucleotide with a ribose sugar having a nucleophilic 2' hydroxyl. The APOBEC3G gene encoding a ssDNA-specific cytidine deaminase could be targeted to tumor stem cells to hypermutate the ssDNA, thereby restricting replication. One skilled in the art would be familiar with the means of construction APOBEC3G expression vectors and delivering them to the target cells.

### Delivery of Agent

In the methods of the application, the agent can be administered by itself, or in a composition (e.g., a pharmaceutical or physiological composition) comprising the agent with a physiologically-compatible carrier or excipient. It can be administered either *in vivo* (e.g., to an individual) or *in vitro* (e.g., to a tissue sample). The methods can be used not only for human individuals, but also are applicable for veterinary uses (e.g., for other mammals, including domesticated animals (e.g., horses, cattle, sheep, goats, pigs, dogs, cats, birds) and non-domesticated animals. The carrier and composition can be sterile. The formulation should suit the mode of administration.

Suitable pharmaceutically acceptable carriers include but are not limited to water, salt solutions (e.g., NaCl), saline, buffered saline, alcohols, glycerol, ethanol, gum arabic, vegetable oils, benzyl alcohols, polyethylene glycols, gelatin, carbohydrates such as lactose, amylose or starch, dextrose, magnesium stearate, talc, silicic acid, viscous paraffin, perfume oil, fatty acid esters, hydroxymethylcellulose, polyvinyl pyrolidone, etc., as well as combinations thereof. The pharmaceutical preparations can, if desired, be mixed with auxiliary agents, e.g., lubricants, preservatives, stabilizers, wetting agents, emulsifiers, salts for influencing osmotic pressure, buffers, coloring, flavoring and/or aromatic substances and the like which do not deleteriously react with the active agents.

Methods of introduction of these compositions include, but are not limited to, intradermal, intramuscular, intraperitoneal, intraocular, intravenous, subcutaneous, topical, oral and intranasal. Other suitable methods of introduction can also include rechargeable or biodegradable devices, particle acceleration devises ("gene guns") and slow release polymeric devices. The pharmaceutical compositions can also be administered as part of a combinatorial therapy with other agents.

The composition can be formulated in accordance with the routine procedures as a pharmaceutical composition adapted for administration to human beings or animals. For example, compositions for intravenous administration typically are solutions in sterile isotonic aqueous buffer. Where necessary, the composition may also include a solubilizing agent and a local anesthetic to ease pain at the site of the injection. Generally, the ingredients are supplied either separately or mixed together in unit dosage form, for example, as a dry lyophilized powder or water free concentrate in a hermetically sealed container such as an ampule or sachette indicating the quantity of active agent. Where the composition is administered by injection, an ampule of sterile water for injection or saline can be provided so that the ingredients may be mixed prior to administration.

Thus, as a result of the present invention, assay methods are now available to identify novel therapeutic agents suitable for use in inhibiting the replication of bell-shaped nuclei of tumor and fetal stem cells. Further, novel therapeutic methods are also available for treating preneoplasias and neopleasias associated with tumor stem cells with bell-shaped nuclei.

The following examples are provided to illustrate the resent invention and are not intended to be limiting in any way.

### EXAMPLES:

A description of examples follows.

### Example 1. Establishing a source of tissues and tumors.

Adult tissue and tumor specimens were obtained as surgical discards by and from collaborators at the Massachusetts General Hospital, Department of Pathology (Gostjeva, E. et al., Cancer Genet. Cytogenet., 164:16-24, 2006). Use of anonymous discarded tumor and tissue sections has been approved by the MIT Committee on Use of Humans as Experimental subjects through the laboratory of Prof. W. G. Thilly.

### Development of a method for tissue excision, fixation, spreading and DNA staining.

The following protocol permits visualization of nuclei of tissue and tumor specimens of desirable clarity for structural and quantitative observations of chromosomes and nuclei. Key elements are use of fresh tumor samples fixed within 30 minutes of surgery and avoidance of standard procedure of thin sectioning. The bell-shaped nuclei are apparently early victims of autolysis in tissue and tumor samples and are no longer discernable some 45 minutes after resection. Standard 5 micron sections simply slice through the several nuclear forms discovered nearly all of which have minimum diameters greater than 5 microns. The specific technique devised is as evidence of significant progress:
Within 30 minutes after resection sheets (~1 cm²) such as stripped colonic mucosa or ~1mm thick sections of adenomas, adenocarcinomas or metastases are placed in at least three volumes of freshly prepared 4°C Carnoy's fixative (3:1, methanol: glacial acetic acid). Fresh fixative is replaced three times (every 45 minutes) and then replaced by 4°C 70% methanol for sample storage at -20°C. Fixed sections are rinsed in distilled water and placed in 2 mL of 1N HCl at 60°C for 8 minutes for partial hydrolysis of macromolecules and DNA depurination. Hydrolysis is terminated by rinsing in cold distilled water. The rinsed sample is steeped in 45% acetic acid (room temperature) for 15 to 30 minutes for "tissue maceration" that allows spreading and observation of plant and animal tissue sections with gentle pressure on microscope cover slips. Each macerated section is bisected into ~0.5 x 1mm pieces and transferred with 5 µL of acetic acid to a microscope slide under a cover slip. For tissue spreading 5 layers of filter paper are placed on the cover slip. A tweezers handle is moved steadily in one direction along the filter paper with slight and even pressure. In well-spread colonic tissue there are no damaged nuclei while crypts are pressed into what is essentially a monolayer. Cover slips are removed after freezing on dry ice and slides are dried for one hour. Slides are placed in Coplin jars filled with Schiff's reagent to stain partially depurinated DNA (Feulgen staining) at room temperature for one hour, rinsed in the same Coplin jar two times with 2xSSC (trisodium citrate 8.8 g/L, sodium chloride 17.5 g/L), once for 30 seconds and once quickly. Slides are then rinsed with distilled water and are suitable for image analyses of nuclei (Gostjeva, E., Cytol. Genet., 32:13-16, 1998). To achieve superior resolution, slides are further stained with Giemsa reagent. Immediately after rinsing in 2xSSC slides are placed in 1% Giemsa solution (Giemsa, Art. 9204, Merck) for 5 minutes then rinsed quickly, first in Sörenssen buffer (disodium hydrogen phosphate dihydrate 11.87 g/L, potassium dihydrogen phosphate 9.07 g/L), and then distilled water. The slides are dried at room temperature for one hour and placed in a Coplin jar filled with xylene for at least 3 hours to remove fat. Cover slips are glued to the slides with DePex mounting media and dried for 3 hours prior to high resolution scanning.

Alternatively, maceration can be achieved by exposure to proteolytic enzymes such as, for example, collagenase II, to achieve isolation of live cells with a defined nuclear morphotype.

### Microscope and image processing system.

The software for quantitative image analysis that is used herein utilizes an approach to background suppression adapted from earlier satellite surveillance systems. This technology was acquired by the Kontron corporation in Germany that has since been itself acquired by Zeiss, Inc. All images have been obtained using a customized *KS-400 Image Analysis System*™*, Version 3.0,* (Zeiss, Germany) consisting of a motorized light microscope, Axioscope™, color CCD camera, AxioCam™ (Zeiss, Germany) linked to a personal computer. Images are transmitted from the microscope at 1.4/100 magnification of the planar apochromatic objective using visible light and a *560 nm* (green) filter when Feulgen stain alone was employed. No filter is used when Feulgen-Giemsa staining is employed. The frame grabber and optimal light exposure are adjusted prior to each scanning session. Nuclear images are recorded at a pixel size 0.0223 x 0.0223 microns.

### Embryonic gut.

Seven distinct nuclear morphotypes (large spheroid, condensed spheroid, ovoid, bean-, cigar-, sausage- and bell-shaped) were found throughout the fetal gut samples (FIG. 1A). The bell-shaped nuclei that seemed to be held open by condensed chromatin resembling condensed chromosomes (FIG. 1B). Bell-shaped nuclei were organized in a linear 'head -to- toe' orientation within ~ 20-50 micron tubes, or syncytia (FIG. 2). The 'head-to-toe' pattern of the bell-shaped nuclei was preserved in all embryonic tubes observed but tubes snaked backwards and forwards such that parallel tubes had locally anti-parallel orientation of bell-shaped nuclei.

Bell-shaped nuclei were observed to undergo symmetrical and asymmetrical amitoses but only within the syncytia (FIG. 3). Symmetrical amitoses of bell-shaped nuclei resembled a simple separation of two stacked paper cups. At the highest resolution, condensed chromatin resembling paired chromosomes appeared to form an annulus that maintained the bell "mouth" in an open condition. Outside of the tubular syncytia mitoses were frequently observed for each of the several "closed" nuclear morphotypes and small colonies were evident consisting of cells of identical nuclear morphotype. Specific "closed" nuclear morphology was preserved in early prophase as shown in FIG. 1.

### Normal colonic epithelium.

Nearly all nuclei in crypts could be observed from the crypt base to the luminal surface (FIG. 4A). Many crypts spread in such a way that individual nuclear shapes could be discerned. Cells with ovoid or spheroid nuclei line the crypt from just above the base to the epithelial extension into the lumen. (FIG. 4C). In the first ~2⁵ cells of the crypt base, a potentially distinct, ninth nuclear morphotype predominated that may be characterized as discoid, ~2-3 microns thick and ~ 10 microns diameter (FIG. 4B). In less than 1% of all crypt bases in which the cells were well separated a solitary bell-shaped nucleus was discerned among the apparently discoid nuclei (FIGS. 4A and 4B). A similar low frequency of bell-shaped nuclei has been observed in preparations of adult liver. In an adult colon without any pathological indication of neoplasia or preneoplasia no other nuclear morphological variant was observed in a cell-by-cell scan of more than a thousand well spread crypts.

### Adenomas.

Adenomas contained many crypts, indistinguishable from normal colonic crypts each with ~ 2000 cells. These were frequently found in branching forms as shown in FIG. 5A. The same spheroid and ovoid nuclei in the crypt walls as in the normal colonic crypts but more frequently than in the normal colon there were one or two bell-shaped nuclei in the crypt base. Irregular lobular structures were also observed containing up to ~ 8000, cells the cells of which were more easily spread by tissue maceration. In nearly all of the irregular structures there were two or more bell-shaped nuclei oriented with the bell openings in the direction of the body of the structure (FIG. 5B). In addition many diverse cells and groups were interspersed among the crypts and irregular structures (FIG. 5C). Some regular structures appeared to be growing toward full-sized normal crypts containing -250, ~500 or ~1000 cells. Many cell groups were seen as "rings" of exactly 8, 16, 32, 64 and 128 cells each with one bell-shaped nucleus (FIG. 5D).

Higher magnification examination revealed that while most of the cells of the walls of the crypt-like structures had spherical or ovoid nuclei as in the normal adult colonic crypt. Colonies of cells with either ovoid, cigar- or bullet-shaped nuclei appeared in the irregular lobular structures suggesting a fusion of several different colonies. Colonies with ovoid and cigar-shaped nuclei had been observed in embryonic hindgut but the bullet-shaped nuclear morphotype was seen only in adenomas and adenocarcinomas (FIG. 5E). The bullet-shaped nuclear morphotype also arose from bell-shaped nuclei by asymmetrical amitoses with the irregular end emerging first. Small colonies of cells with bullet-shaped nuclei were seen and these colonies contained cells undergoing ordinary mitoses save for the interesting fact that the peculiar nuclear morphologies were retained to some extent from prophase through anatelophase.

While rare in the normal adult colon, the bell-shaped nuclei appeared frequently and in a number of adenoma contexts. Some were found as one to ten or more "bells" in the spaces among the crypt-like structures (FIG. 5D). Others were found as single "bells" in multicellular ring structures in which one bell nucleus was always seen in the ring with (2ⁿ - 1) cells of spheroid or other morphotype (FIGS. 5C and 5D).

Bell-shaped nuclei appeared as single bells, more often as a pair of bells or occasionally 4 or 8 bells within the crypt-like structures basal cup. In the much larger irregular lobular structures, bell-shaped nuclei were anatomically integrated into the walls of the aberrant structures mixed with cells of other nuclear morphologies. It appeared as if these larger irregular crypt-like structures were mosaics of multiple different kinds of clusters each with it's own nuclear morphotype. Large adenomas (~1 cm) were estimated to contain about 1000 bell-shaped nuclei. Hundreds bell-shaped nuclei have been observed in each of multiple adenomas but not a single bell-shaped nucleus in any adenoma has been observed in the symmetrical form of nuclear fission frequently found in embryonic sections; several examples of asymmetrical nuclear fission have been observed however, in adenomas.

### Adenocarcinomas.

Adenocarcinomas like adenomas contained the admixture of crypts, larger irregular structures and inter-cryptal clusters of 16, 32, 64 and 128 cells. Bell-shaped nuclei were still found as singlets, pairs or larger numbers in the basal cup of crypts and embedded in complex whorls in the walls of the larger irregular lobular structures (FIG. 6). The set of nuclear morphotypes in the adenocarcinomas appear to be identical with the set seen in adenomas including the bullet-shaped morphotype.

A discernible difference between adenomas and adenocarcinomas was that the crypt-like structures were randomly oriented with regard to the tumor surface. Also crypts and irregular structures were not found frequently in the tumor interior, which may be better characterized as an eclectic but not chaotic collection of smaller, locally organized structures.

The most noticeable difference by which the adenocarcinomas differed from adenomas was the frequent appearance of apparently organized groupings of more than hundreds of bell-shaped nuclei many of which were frequently (~1%) involved in symmetrical nuclear fissions. These symmetrical fissions were later identified as comprising condensed nuclear material. A bell-shaped nucleus would have an amount of DNA equal to that of a normal haploid cell. As the bell-shaped nuclei begin to undergo the "cup-from-cup" symmetrical division, the DNA content increases to 1.05 the amount of DNA contained in a haploid genome (approximately the increase one expects if the centromeres are replicated). The DNA content remains at this level until much later in the "cup-from-cup" process at which point the two nuclei contain 2 times the amount of DNA material. It is during the stage when perhaps only the centromeres have replicated, and the strands of the genome are separated that the genome is organized primarily into ssDNA. Not until replication much later in the process does the genome become dsDNA again.

At low magnification these structures appeared in the spaces among crypt-like structures and looked like a spider web or leaf vein skeleton. At higher magnification the thin veins were found to be partially ordered strands of cells with bell-shaped nuclei having the curious characteristic of having their mouths oriented in the same direction, 90° from the vein axis (FIG. 6C). Bell-shaped nuclei were also found in locally delimited syncytia in the 'head-to-toe' orientation (FIG. 6C) observed in the embryonic gut but not in the adenomas. Millions of bell-shaped nuclei are estimated to be in an adenocarcinomatous mass with frequent symmetrical and asymmetrical amitoses (FIGS. 6D and 6E). Metastases of colorectal tumors to the liver recreated the pattern of nuclear morphotypes, crypts and irregular structures seemingly indistinguishable from those observed for adenocarcinomas.

### Confocal microscopy on 3D preserved single bell-shaped nuclei and pairs of symmetrically dividing bell-shaped nuclei.

To perform *confocal microscopy* on 3D preserved single bell-shaped nuclei and pairs of symmetrically dividing bell-shaped nuclei, DeltaVision^{®} RT Restoration Imaging System at Imaging Center, Whitehead Institute is used. The system provides real-time 2D deconvolution and 3D Z projections for restoration of nuclei images.

Counterstaining of nuclear cytoplasm (FITC-phalloidin) and nuclear (DAPI) have been applied to explore the interior structure of the bell-shaped nuclei. The cells are spread on the slide following same procedure as for Feulgen staining: by 'hydrolysis' maceration. The difference is that fixations in two different fixatives is applied to compare the results: Camoy's fixative (4°C) and 3.7% formaldehyde for 15 min and blocking solution for 2 hours in 2% BSA (2g), 0.2% nonfat milk (0.2g), 0.4% triton X-100 (400 µL) in 100 mL of PBS (room temperature), the latter as recommended for fixations of live tissue cells. Microscopic slides with tissue spreads on it, after twice washing in PBS, are transferred to humidity chamber, 100 mL droplets of primary antibodies diluted appropriately in blocking solution are dropped to cover the entire area of the spread and coverslips are sealed on the top by rubber cement, placed into container wrapped in foil and placed in the humidity chamber in the cold room overnight. Unsealed slides then washed three times in PBS. The slides are taken out and 100µL droplets of secondary antibodies and/or cell stains *(e.g.,* FITC-phalloidin, DAPI) diluted appropriately in blocking solution are placed again to cover the area containing the cells spread and transferred to humidity chamber placed in container. The container/humidity chamber is sealed, wrapped in foil and placed at room temperature for 2 hours. Slides are washed five times in PBS and prepared in a way that each have 2-5 µL droplets of mounting media (anti-fades *SlowFade, VectaSheild* or *ProLong).* Coverslips are mounted making sure an excess PBS is removed (dabbing the corner of the coverslip on a paper towel). The number of bubbles formed during mounting are limited by introducing the edge of the coverslip into the mounting media prior to lowering it completely. Coverslip are sealed on the slide using nail polish and the slides stored in dark at 4°C (or -20°C for longer periods). The slides are visualized using DeltaVision^{®} RT Restoration Imaging System.

The protocol of Feulgen-Schiff procedure, which has been demonstrated to be accurate for the cytochemical localization of DNA and stoichiometry, was used to measure nuclei DNA contents. The DNA content was measured in single nuclei by measuring absorbance of molecules of a Feulgen-DNA (dye-ligand) complex (Kjellstrand, P., J. Microscopy, 119:391-396, 1980; Andersson, G. and Kjellstrand, P., Histochemie, 27:165-200, 1971). Non-dividing (interphase) and dividing bell-shaped nuclei were measured by measuring optical density integrated over the entire area (IOD) of each individual nucleus using software adapted from *KS 400* image analysis system *(Zeiss Inc, Germany).*

This particular image analysis workstation (See FIG. 9D) consists of a microscope Axioscop 2 MOT (Zeiss) coupled with AxioCam color CCD camera (Zeiss) connected to a computer, assembled by Carl Zeiss Inc. engineers, is capable of high-resolution image microscopy of nuclear and cell structures that is about 1000 bp of DNA per pixel in early prophase chromosomes measurements. Therefore, accurate measurements of condensed chromatin domains of ~ 1 Mb pairs in interphase nuclei are possible. Images of the were scanned under constant parameters of magnification, light exposure and thresholding (contouring) of the nuclei using 560 nm green filter. This way of DNA content measurement was chosen as promising the most accurate results (Biesterfeld. S. et al., Anal. Quant. Cytol. Histol., 23:123-128, 2001; Hardie, D. et al., J. Histochem. Cytochem., 50:735 - 749, 2002; Gregory and Hebert, 2002; Gregory, 2005).

Fluorescent *in situ* Hybridization to define the spatial distribution of all 24 human chromosomes in bell-shaped nuclei in interphase and during nuclear fission.

FISH was used to determine the whole chromosomes are involved in condensation that appears as a 'ring' on the top of the bell-shaped nuclei. Basically, labeling of chromosomes in the 'ring' is foreseen as a means to analyze their transformation when bell-shaped nuclei gives rise to a nucleus of different morphology (as shown in FIG. 10B) as well as developing of a fluorescence marker to recognize these nuclei by other means rather then nuclear morphology.

Tumor cells of not more then 1-5 x 10⁷ cells per slide are spread on the slide. The slides fixed in two different ways of cells spreading: one used in protocol for Feulgen DNA image cytometry and another proposed by Gibson for isolation of epithelial cells from colonoscopic biopsy specimens (Gibson, P. et al., Gastroenterology, 96:283-291, 1989). The latter is basically taking a tumor tissue within 30 min of surgery and immediately placing it in 50 mL of cold Hank's balanced salt solution, then washed. The specimens are then minced with a scalpel blade and digested for 1.5 h in 4 mL of collagenase-Dispase medium (culture medium containing 1.2 U/ml Dispase I *(Boehringer Mannheim Biochemicals, Indianapolis, Ind.*) and 50 U/ml collagenase type IV (Worthington, Biochemical Corp., Freehold, N.J.). The pellet is spread on the surface of microscopic slide by gentle sliding pressure on the coverslip. The spreading by 'hydrolysis' maceration serves as positive control to check if any distortion of bell-shaped nuclear morphology has occurred after applying collagenase-Dispase treatment for cells spreading. Prepared slides are dried out and put at 37°C overnight. Slides then dehydrated sequentially in ice cold 70%, 80%, at room temperature 100% ethanol for 2 minutes each and dried completely, undergo denaturation in 70% formamide/2xSSC at 72°C for 2 minutes and immediately dehydrated again with the same sequence and dried completely. Hybridization mixtures prepared that contains 7µL hybridization buffer, 2µL sterile water, and 1µL probe. Mixtures are denatured at 72°C for 8 to 12 minutes and immediately added to slides which then coverslipped, sealed with rubber cement, and put at 37°C in a dark, humidified box overnight.

Slides are then dehydrated in cold 70% ethanol, cold 80% ethanol, and room temperature 100% ethanol for 2 minutes each; denatured in 70% formamide, 2xSSC at 72°C for 50-60 seconds, depending on the extent of acetic acid denaturation. Slides are dehydrated again in cold 70% ethanol, cold 80% ethanol, and room temperature 100% ethanol for 2 minutes each. The hybridization mix includes 7 µL hybridization buffer, 1.5 µL sterile H₂O, and 1.5 µL. Whole Chromosome Paint probes (*Vysis*) with either Spectrum Orange or Spectrum Green fluorescent dye is applied. Hybridization mix is denatured for 5-10 minutes at 72°C and slides subsequently dried completely. Hybridization mix is applied to the slides, coverslipped and sealed with rubber cement. Slides are then incubated overnight at 37°C in a humidified box. On the following day, slides are washed in 50% formamide, 2xSSC at 42°C twice for 8 minutes each. Slides are then washed with 2xSSC at 37°C for 8 minutes and then washed three times in 1xPBD (0.05% Tween, 4xSSC) at room temperature for 1 minute each. Then 10 µL DAPI II Antifade, 125ng/mL (Vysis) and coverslips is added. The excess DAPI II Antifade is blotted away and the slides sealed with rubber cement. Slides are kept in the dark at -20°C prior to image scanning procedure.

### Use of quantitative DNA cytometry to track DNA synthesis before, during and after nuclear fission of bell-shaped nuclei.

The techniques described herein permit detection of differences as low as 2% between any two nuclei or the anatelophases of sister nuclei during mitosis in human cell cultures. These techniques were used to determine when DNA is synthesized by cells or syncytia containing bell-shaped nuclei. This involved scanning nuclei that appear to be in the process of nuclear fission. It is noted that in general fetal bell-shaped nuclei containing the expected amount of DNA of a diploid human cell by comparison to human lymphocyte DNA content on the same stained slide. In addition, it is noted that the amount of DNA in bell-shaped nuclei of human preneoplastic lesions and tumors betrays a wide variation around a mean that is on average greater than the diploid DNA amount. Measurements have revealed another totally unexpected finding: DNA synthesis is concordant with rather than preceding the process of nuclear fission for both symmetrical and asymmetrical nuclear fissions involving bell-shaped nuclei. Nuclei appear to be well along in the process of 'cup-from-cup' separation before an increase in total DNA content from the single nucleus amount is clearly detected. The total amount of DNA increases from a low value approximating the average of single tumor nuclei in nuclei apparently beginning fission and reaches about two times the average nuclear content in nuclei that appear to have just completed fission.

### Example 2. Syncytial bell-shaped nuclei in fetal organogenesis.

A series of previously unrecognized nuclear forms were identified in human fetal preparations that give rise to the bell-shaped nuclei. These forms were detected in the fifth week, as were the first tubular syncytia, which contain bell-shaped nuclei. Examples of these are shown in FIGS. 13A-D. This as an important finding marking the morphological transition from mitotic, spherical nuclei of early embryogenesis to the later amitotic, bell-shaped nuclei that represent the generative "stem" cell lineage of net growth and differentiation.

These findings are consistent across tissue types, as they have been observed in a series of tissue preparations including, for example, muscle, developing limbs, nervous tissue and visceral organs including the stomach, pancreas, bladder, lung and liver. The syncytia are found as clusters of~16-24 syncytia regularly spaced within the developing organ mass, each with ~16 bell-shaped nuclei. Syncytia are apparent in the least developed human material available (~5 weeks) and have disappeared by the thirteenth week. After the twelfth week the bell-shaped nuclei are regularly distributed in three dimensions in a manner peculiar to each organ.

FIG. 13A shows a nucleus with a condensation of~10% of the total DNA content as a "belt" around the long axis of spherical or slightly oval nuclei. FIG. 13B shows a nucleus in which two condensed nuclear "belts" appear to have separated but are still part of a single nucleus. FIG. 13C shows a pair of nuclei that appear to have arisen by fission of the two-belted nucleus of FIG. 13B. FIG. 13D shows that each syncytium contains a set of bells with a single pair of bells at its linear midpoint with mouths facing as in FIG. 13C. These images suggest to us that a series of symmetrical divisions create nuclei pushing away from a central pair. Syncytial structure is detected in groups as small as four bell-shaped nuclei.

In studies of the nuclear morphotypes of carcinogenesis, nuclei showed similar belts- one or two around the long axis of oval nuclei - in small numbers in colonic adenomas (FIG. 14A) and adenocarcinomas (FIG. 14B). This finding confirms and extends support for the general hypothesis that oncogenesis shares many key phenotypic transitional steps of ontogenesis presenting, however, in reverse order of appearance.

### FISH staining specific for human centromeres.

Extra-syncytial bell-shaped nuclei actually contain human DNA. Most centromeres are associated with the region of condensed DNA at the mouth of the bell-shaped nuclei in fetal samples. Interestingly, standard FISH protocols do not stain intra-syncytial bell-shaped or other shaped nuclei suggesting that the contractile element-containing sheath of the syncytium may block entry of the FISH reagents. FIG. 15 shows centromeres (in green) in spherical (FIG. 15A), "cigar"-(FIG. 15B) and bell- (FIG. 15C) shaped nuclei from tissues of human 12 weeks fetal colon.

It was also observed that the DNA content of sister nuclei are equal in fetal amitoses of bell-shaped nuclei, but they betray a marked degree of unequal DNA segregation in amitoses of bell-shaped nuclei in human tumors from multiple tissues of origin. Although no examples of amitotic fission among the bell-shaped nuclei of colonic preneoplastic polyps have been found, it is noted that the marked dispersion of DNA content among the dozens of bell-shaped nuclei found per polyp suggests that unequal DNA partitioning is a phenomena that is operative in preneoplasia as well as neoplasia, but not in fetal fissions of bell-shaped nuclei. These observations extend the observations of Virchow and Cohnheim that tumor tissue and embryonic tissue have similar histological features while also extending those of Boveri that tumor cells in mitosis betray a large fraction of aberrant chromosomes common to all cells of the tumor- suggesting an earlier common origin in unstable chromosomal formation or segregation.

### Nuclear Morphology in mice.

All of the various forms of nuclei, in particular including the bell-shaped nuclei, pre-syncytial and syncytial forms in morphology almost identical to FIGS. 13A-D were found in tissue of fetal mice with the presyncytial forms first detected in 12.5 day, then in 14.5 - 16.5 days fetuses closely paralleling the period of organ definition in the fetal mouse. While these findings in the mouse are not surprising given the human observations, they open up a wide spectrum of possibilities of studies of organogenesis in non-human species not ethical or possible in humans.

In samples of quality fixed fetal discards ranging from the fifth through the sixteenth week of gestation, syncytia are no longer evident but bell-shaped nuclei are distributed in regular patterns throughout the growing organs.

### Example 3. Stem Cell Markers

Abundant syncytia and bell-shaped nuclei of the primitive gut are used to apply a series of histochemical procedures including FISH for defining the positions of chromosomes and chromosomal elements, various contractile molecules (*e.g*., actin) and other identifiable markers including those commonly denominated "stem cell markers". Techniques described herein are applied to the task of collecting syncytia and individual nuclei using the ZEISS-P.A.L.M. microdissection instrument. The criterion of success is the collection of a series of samples homogeneous with regard to syncytial forms or nuclear morphotypes in numbers equal to or larger than 10,000 nuclear equivalents, numbers sufficient for scanning of cellular mRNAs, most common proteins and glycosaminoglycans.

### Example 4. Acridine Orange Staining

Additional data to identify the replicative intermediate configurations are shown in FIGS. 16-19. FIG. 18B is of particular importance. It conclusively demonstrates the presence of a ssDNA genome within the bell-shaped nuclei. An Acridine orange stain is utilized, which fluoresces red specifically and solely upon binding to ssDNA.

Prior to staining slides were exposed to RNase at concentration 2 mg/mL for 2 hours at 37° C in water bath. Slides were washed in EDTA for I minute, rinsed in molecular grade water and immediately proceed to staining in acridine orange solution according to protocol described in Bertalanffy (Ann New York Sci, 84: p. 227-238 (1960); Ann New York Sci 93:16: p. 717-747 (1962)). Briefly, acridine orange stain was 0.05 gm diluted in 500.0 ml distilled water and 5.0 ml of acetic acid added. Room temperature solution was checked for pH being in a range 3.1 - 3.4 each time before placing slides into a Coplin jar in a dark, room temperature. Slides were stained for 30 minutes, rinsed in 0.5% acetic acid in 100% alcohol, in 100% alcohol, in PBS and visualized under fluorescent microscope while still wet with cover-slip on.

### Example 5. ssDNA Antibody Staining

The monoclonal antibody Mab F7-26 was used for detecting ssDNA intermediates (Bender MedSystems GmbH, Vienna, Austria). The stock solution of antibody was diluted 10x (with PBS and 5% foetal bovine serum) before use. Series of washing steps were performed prior to applying ssDNA antibody, using 0.005% pepsin in 10 mM, HCl 50 mM MgCl2 and 1% formaldehyde in 50 mM MgCl2 (described in details, Fomina et al., 2000). Following each wash step, slides were washed 3x with PBS (5 minutes each). Finally slides were washed with 0.05% Tween, then with blocking protein 3% BSA in Tween and again in 0.05% Tween. Afterwards, slides were treated with Mab F7-26 in 5%FBS for 60 minutes at room temperature, followed by applying anti-mouse Ig-G-FITC, Alexa 488 for 30 minutes. Slides were stained with DAPI, dehydrated in a series of ethanol, 70%, 90% and 100%, air-dried and mounted in 'Citiflour' media.

It appears that the double dsDNA rings at the "mouth" of the bell -shaped nuclei are first segregated into two ssDNA rings and these are copied to create the four dsDNA rings previously observed as the beginning of bell segregation and DNA increase using Feulgen staining and quantitative cytometry.

It is notable that in the fetal metakaryotic syncytial phase that in any tissue sample only some 5-15% of syncytia display ssDNA but in those synctia with at least one nucleus displaying ssDNA many others are usually observed extending our earlier observations that bell-shaped nuclei in syncytia segregate and synthesize DNA synchronously. Based on the growth rates of fetuses and the fraction of nuclei betraying any ssDNA, we have made a preliminary estimate of ~8 hours four the DNA segregation/synthetic period with the period of separated ssDNA masses being somewhat less than half this period. See FIGS. 18A-F.

While this invention has been particularly shown and described with references to preferred embodiments thereof, it will be understood by those skilled in the art that various changes in form and details may be made therein without departing from the scope of the invention encompassed by the appended claims.

## Claims

1. A method for identifying an agent that inhibits tumor growth, comprising
a) contacting a tumor from a non human subject or non human host animal with a candidate agent, wherein the tumor comprises tumor stem cells comprising bell-shaped nuclei, and wherein some or all of the bell-shaped nuclei are in a replicative intermediate configuration associated with ssDNA;
b) maintaining the tumor in contact with the candidate agent under conditions suitable for the agent to interact with the tumor;
c) excising a sample of the tumor and determining the presence of, absence of, or amount of ssDNA in a replicative intermediate configuration in bell-shaped nuclei, in the tumor sample after the contact in step b); and
d) comparing the presence of, absence of, or amount of ssDNA in a replicative intermediate configuration in bell-shaped nuclei, in the tumor sample after contact with the candidate agent to the presence of, absence of, or amount of ssDNA in a replicative intermediate configuration in bell-shaped nuclei in a control sample,
whereby a reduction in the amount of, or absence of ssDNA in a replicative intermediate configuration in bell-shaped nuclei, in the sample after contact with the candidate agent indicates that the agent inhibits tumor growth.

2. The method of Claim 1, wherein the tumor sample is obtained from a solid tumor resulting from a xenograft into a host organism.

3. The method of Claim 1, wherein ssDNA is detected by staining the sample with acridine orange and visualizing the ssDNA.

4. The method of Claim 1, wherein the candidate agent
a) targets single-stranded DNA, preferably wherein the candidate agent
b) disrupts annealing of DNA, degrades single-stranded DNA, or disrupts DNA replication from a single-stranded DNA template.

5. The method of Claim 1 wherein, in step c), prior to staining the sample is exposed to RNase at a concentration of 2mg/ml for 2 hours at 37°C in a water bath, washed in EDTA for 1 minute, rinsed in molecular grade water and immediately stained in acridine orange solution: 0.05g acridine orange diluted in 500.0ml distilled water and 5.0ml of acetic acid, pH at room temperature being in a range of 3.1-3.4, samples placed into a Coplin jar in the dark at room temperature, stained for 30 minutes, rinsed in 0.5% acetic acid in 100% alcohol, in 100% alcohol, in PBS before visualisation under fluorescence microscope while still wet with coverslip on.

## Patentansprüche

1. Verfahren zum Identifizieren eines Stoffs, der das Tumorwachstum hemmt, das umfasst:
a) Inkontaktbringen eines Tumors eines nichtmenschlichen Versuchsobjekts oder nichtmenschlichen Wirtstiers mit einem Kandidatenstoff, wobei der Tumor Tumorstammzellen umfasst, die glockenförmige Nuclei enthalten, und wobei einige oder alle glockenförmige Nuclei in einer replikativen Zwischenstruktur vorliegen, die mit ssDNA verbunden ist;
b) Inkontakthalten des Tumors mit dem Kandidatenstoff unter Bedingungen, die für eine Wechselwirkung des Stoffs mit dem Tumor geeignet sind;
c) Herausschneiden einer Probe des Tumors und Feststellen des Vorhandenseins oder der Abwesenheit von ssDNA oder der Menge an ssDNA in einer replikativen Zwischenstruktur in glockenförmigen Nuclei in der Tumorprobe nach dem Kontakt in Schritt b); und
d) Vergleichen des Vorhandenseins oder der Abwesenheit von ssDNA oder der Menge an ssDNA in einer replikativen Zwischenstruktur in glockenförmigen Nuclei in der Tumorprobe nach dem Kontakt mit dem Kandidatenstoff mit dem Vorhandensein oder der Abwesenheit von ssDNA oder der Menge an ssDNA in einer replikativen Zwischenstruktur in glockenförmigen Nuclei in einer Kontrollprobe,
wobei eine Verringerung der Menge an oder die Abwesenheit von ssDNA in einer replikativen Zwischenstruktur in glockenförmigen Nuclei in der Probe nach dem Kontakt mit dem Kandidatenstoff darauf hinweist, dass der Stoff das Tumorwachstum hemmt.

2. Verfahren nach Anspruch 1, wobei die Tumorprobe von einem festen Tumor erhalten wird, der von einem Xenograft in einen Wirtsorganismus herrührt.

3. Verfahren nach Anspruch 1, wobei ssDNA durch Färben der Probe mit Acridinorange und Sichtbarmachen der ssDNA nachgewiesen wird.

4. Verfahren nach Anspruch 1, wobei der Kandidatenstoff
a) auf Einzelstrang-DNA abzielt, wobei der Kandidatenstoff vorzugsweise
b) das Annealing der DNA unterbricht, Einzelstrang-DNA abbaut oder die DNA-Replikation von einem Einzelstrang-DNA-Template unterbricht.

5. Verfahren nach Anspruch 1, wobei in Schritt c) vor dem Färben die Probe 2 h bei 37 °C in einem Wasserbad RNase in einer Konzentration von 2 mg/ml ausgesetzt wird, 1 min in EDTA gewaschen wird, in molekular reinem Wasser gespült wird und sofort in einer Acridinorgane-Lösung, 0,05 g Acridinorange verdünnt in 500,0 ml destilliertem Wasser und 5,0 ml Essigsäure, pH-Wert bei Raumtemperatur in einem Bereich von 3,1-3,4, gefärbt wird, wobei die Proben in einer Färbeküvette nach Coplin im Dunklen bei Raumtemperatur gehalten werden, 30 min gefärbt werden, in 0,5 % Essigsäure in 100 % Alkohol, in 100 % Alkohol, in PBS gespült werden vor dem Sichtbarmachen im Fluoreszenzmikroskop, bei dem sie weiterhin im nassen Zustand und mit aufgebrachtem Deckglas vorliegen.

## Revendications

1. Méthode d'identification d'un agent qui inhibe la croissance d'une tumeur, comprenant
a) mettre en contact une tumeur d'un sujet non humain ou d'un animal hôte non humain avec un agent candidat, où la tumeur comprend des cellules souches de tumeur comprenant des noyaux en forme de cloche, et où quelques-uns ou tous les noyaux en forme de cloche sont dans une configuration intermédiaire de réplication associée à l'ADNss;
b) maintenir la tumeur en contact avec l'agent candidat sous des conditions appropriées pour que l'agent interagisse avec la tumeur;
c) exciser un échantillon de la tumeur et déterminer la présence, l'absence ou la quantité de l'ADNss dans une configuration intermédiaire de réplication dans des noyaux en forme de cloche, dans l'échantillon de tumeur après le contact à l'étape b); et
d) comparer la présence, l'absence ou la quantité d'ADNss dans une configuration intermédiaire de réplication dans des noyaux en forme de cloche, dans l'échantillon de tumeur après le contact avec l'agent candidat à la présence, l'absence ou la quantité d'ADNss dans une configuration intermédiaire de réplication dans des noyaux en forme de cloche dans un échantillon de contrôle,
où une réduction dans la quantité, ou l'absence d'ADNss dans une configuration intermédiaire de réplication dans des noyaux en forme de cloche, dans l'échantillon après le contact avec l'agent candidat, indique que l'agent inhibe la croissance de la tumeur.

2. Méthode selon la revendication 1, où l'échantillon de tumeur est obtenu d'une tumeur solide résultant d'une xénogreffe dans un organisme hôte.

3. Méthode selon la revendication 1, où l'ADNss est détecté en colorant l'échantillon à l'orange d'acridine et en visualisant l'ADNss.

4. Méthode selon la revendication 1, où l'agent candidat
a) cible l'ADN à brin simple, de préférence où l'agent candidat
b) rompt l'appariement d'ADN, dégrade l'ADN à brin simple ou rompt la réplication de l'ADN d'un gabarit d'ADN à brin simple.

5. Méthode selon la revendication 1, où à l'étape c), avant la coloration, l'échantillon est exposé à une RNase à une concentration de 2mg/ml pendant 2 heures à 37°C dans un bain d'eau, lavé dans EDTA pendant 1 minute, rincé dans une eau de grade moléculaire et coloré immédiatement dans une solution d'orange d'acridine: 0,05 g d'orange d'acridine dilué dans 500,0 ml d'eau distillée et 5,0 ml d'acide acétique, le pH à la température ambiante étant dans une plage de 3,1-3,4, des échantillons placés dans un pot de Coplin dans le noir à température ambiante, colorés pendant 30 minutes, rincés dans de l'acide acétique à 0,5% dans 100% d'alcool, dans 100% d'alcool, dans du PBS avant la visualisation sous un microscope à fluorescence en étant toujours mouillé, la lamelle étant en place.
